# EUROPEAN PATENT SPECIFICATION

(11) **EP 3 784 786 B1**
(45) Date of publication and mention of the grant of the patent: **12.02.2025**
(21) Application number: 19732756.2
(22) Date of filing: 24.04.2019
(51) Int. Cl.: C12N 15/65, C12N 15/75, C12N 7/00, C12Q 1/14, C12Q 1/70, A61K 35/76

(54) **INDICATOR BACTERIOPHAGE FOR SELECTING AND MONITORING FOR EFFICACY OF THERAPEUTICS AND METHODS FOR USING SAME**
INDIKATORBAKTERIOPHAGE ZUR AUSWAHL UND ÜBERWACHUNG DER WIRKSAMKEIT VON THERAPEUTIKA UND VERFAHREN ZU SEINER VERWENDUNG
BACTÉRIOPHAGE INDICATEUR POUR LA SÉLECTION ET LA SURVEILLANCE DE L'EFFICACITÉ D'AGENTS THÉRAPEUTIQUES ET PROCÉDÉS D'UTILISATION DE CELUI-CI

(30) Priority: 24.04.2018 US 201862661739 P
(43) Date of publication of application: 03.03.2021
(73) Proprietor: Laboratory Corporation of America Holdings, Burlington, North Carolina 27215 (US)
(72) Inventor: GIL, Jose S., Winnetka, California 91306 (US); ERICKSON, Stephen, White Bear Township, Minnesota 55110 (US); NGUYEN, Minh Mindy Bao, Shoreview, Minnesota 55126 (US); ANDERSON, Dwight Lyman, Minneapolis, Minnesota 55406 (US)
(74) Representative: Forresters IP LLP
(86) International application number: PCT/US2019/028967
(87) International publication number: WO 2019/209982

(56) References cited:
- WO-A1-2010/033546
- WO-A1-2017/127434
- LOESSNER M J ET AL: "Evaluation of luciferase reporter bacteriophage A511:luxAB for detection of listeria monocytogenes in contaminated foods", APPLIED AND ENVIRONMENTAL MICROBIOLOGY, AMERICAN SOCIETY FOR MICROBIOLOGY, US, vol. 63, no. 8, 1 August 1997 (1997-08-01), pages 2961 - 2965, XP002966690, ISSN: 0099-2240
- STEVEN HAGENS ET AL: "Reporter bacteriophage A511:: celB transduces a hyperthermostable glycosidase from Pyrococcus furiosus for rapid and simple detection of viable Listeria cells", BACTERIOPHAGE, vol. 1, no. 3, 1 May 2011 (2011-05-01), pages 143 - 151, XP055192917, DOI: 10.4161/bact.1.3.16710
- GOODRIDGE L ET AL: "Reporter bacteriophage assays as a means to detect foodborne pathogenic bacteria", FOOD RESEARCH INTERNATIONAL, ELSEVIER, AMSTERDAM, NL, vol. 35, no. 9, 1 January 2002 (2002-01-01), pages 863 - 870, XP002376541, ISSN: 0963-9969, DOI: 10.1016/S0963-9969(02)00094-7

## Description

### FIELD OF THE INVENTION

This invention relates to methods for the detection of microorganisms using infectious agents.

### BACKGROUND

There is a strong interest in improving speed and sensitivity for detection of bacteria, viruses, and other microorganisms in biological, food, water, and clinical samples. Microbial pathogens can cause substantial morbidity among humans and domestic animals, as well as immense economic loss. Also, detection of microorganisms is a high priority for the Food and Drug Administration (FDA) and Centers for Disease Control (CDC) given outbreaks of life-threatening or fatal illness caused by ingestion of food contaminated with certain microorganisms, e.g., *Staphylococcus* spp.

Traditional microbiological tests for the detection of bacteria rely on non-selective and selective enrichment cultures followed by plating on selective media and further testing to confirm suspect colonies. Such procedures can require several days. A variety of rapid methods have been investigated and introduced into practice to reduce the time requirement. However, these methods have drawbacks. For example, techniques involving direct immunoassays or gene probes generally require an overnight enrichment step in order to obtain adequate sensitivity. Polymerase chain reaction (PCR) tests also include an amplification step and therefore are capable of both very high sensitivity and selectivity; however, the sample size that can be economically subjected to PCR testing is limited. With dilute bacterial suspensions, most small subsamples will be free of cells and therefore purification and/or lengthy enrichment steps are still required.

The time required for traditional biological enrichment is dictated by the growth rate of the target bacterial population of the sample, by the effect of the sample matrix, and by the required sensitivity. In practice, most high sensitivity methods employ an overnight incubation and take about 24 hours overall. Due to the time required for cultivation, these methods can take up to three days, depending upon the organism to be identified and the source of the sample. This lag time is generally unsuitable as the contaminated food, water (or other product) may have already made its way into livestock or humans. In addition, increases in antibiotic-resistant bacteria and biodefense considerations make rapid identification of bacterial pathogens in water, food and clinical samples critical priorities worldwide.

Therefore, there is a need for more rapid, simple and sensitive detection and identification of microorganisms, such as bacteria and other potentially pathogenic microorganisms.

### SUMMARY

The invention is defined in the claims. Embodiments of the invention comprise methods for the detection of microorganisms. The invention may be embodied in a variety of ways.

The invention comprises a recombinant bacteriophage comprising an indicator gene inserted into a late gene region of a bacteriophage genome. The recombinant bacteriophage is a genetically modified *Staphylococcus aureus-specific* bacteriophage genome. The *Staphylococcus aureus* is methicillin-resistant *S. aureus* (MRSA). For example, in certain embodiments the recombinant bacteriophage is a genetically modified bacteriophage genome. In some embodiments, the bacteriophage used to prepare the recombinant bacteriophage specifically infects methicillin-resistant *Staphylococcus aureus* (MRSA). In an embodiment, the recombinant bacteriophage can distinguish *Staphylococcus* in the presence of more than 100 other types of bacteria.

In some embodiments of recombinant indicator bacteriophage, the indicator gene can be codon-optimized and can encode a soluble protein product that generates an intrinsic signal or a soluble enzyme that generates signal upon reaction with substrate. Some recombinant bacteriophage further comprise an untranslated region upstream of a codon-optimized indicator gene, wherein the untranslated region includes a bacteriophage late gene promoter and a ribosomal entry site. In some embodiments, the indicator gene is a luciferase gene. The luciferase gene can be a naturally occurring gene, such as Oplophorus luciferase, Firefly luciferase, Lucia luciferase, or Renilla luciferase, or it can be a genetically engineered gene such as NanoLuc.

Other aspects of the invention include methods for detecting bacteria, such as *Staphylococcus* in a sample, including steps of incubating the sample with a recombinant bacteriophage derived from *Staphylococcus-specific* bacteriophage and detecting an indicator protein product produced by the recombinant bacteriophage, wherein positive detection of the indicator protein product indicates that *Staphylococcus* is present in the sample. In some embodiments, the invention includes methods for the detection of *S. aureus* using a recombinant bacteriophage derived from *S. aureus.* The sample can be a food, environmental, water, commercial, or clinical sample.

In some embodiments of methods for detecting bacteria, the sample is first incubated in conditions favoring growth for an enrichment period of 18 hours or less, 17 hours or less, 16 hours or less, 15 hours or less, 14 hours or less, 13 hours or less, 12 hours or less, 11 hours or less, 10 hours or less, or 9 hours or less, 8 hours or less, 7 hours or less, 6 hours or less, 5 hours or less, 4 hours or less, 3 hours or less, or 2 hours or less. In some embodiments, the total time to results is less than 20 hours, less than 19 hours, less than 18 hours, less than 17 hours, less than 16 hours, less than 15 hours, less than 14 hours, less than 13 hours, less than 12 hours, less than 11 hours, less than 10 hours, less than 9 hours, less than 8 hours, less than 7 hours, or less than 6 hours. In some embodiments, the ratio of signal to background generated by detecting the indicator is at least 2.0 or at least 2.5. In some embodiments, the method detects as few as 1, 2, 3, 4, 5, 6, 7, 8, 9, 10, 15, 20, 30, 40, 50, 60, 70, 80, 90, or 100 of the specific bacteria in a sample of a standard size for the food safety industry.

In another embodiment, the invention may comprise: a method for selecting a treatment for a subject comprising: (i) providing a biological sample obtained from the subject; (ii) detecting a specific microorganism or category of microorganisms in the biological sample using an indicator phage, wherein the indicator phage infects methicillin-resistant *Staphylococcus aureus*(MRSA) and the detection comprises the steps of: (a) incubating the sample with at least one antibiotic to allow for enrichment of bacteria that are resistant to the antibiotic, (b) incubating the enriched samples with bacteriophage that infects the bacterium of interest, wherein the bacteriophage comprises an indicator gene such that expression of the indicator gene during bacteriophage replication following infection of the bacterium of interest results in production of a soluble indicator protein product; and (c) detecting the indicator protein product, wherein positive detection of the indicator protein product indicates that the antibiotic-resistant bacterium of interest is present in the sample; and (iii) selecting a treatment based on the identity of a specific microorganism detected in the biological sample.

In another embodiment, the invention may comprise, a method for monitoring the efficacy of a treatment for a subject having a pathogenic medical condition comprising: (i) providing a biological sample obtained from the subject; (ii) detecting a specific microorganism or category of microorganisms in the biological sample using an indicator phage, wherein the indicator phage infects methicillin-resistant Staphylococcus aureus (MRSA) and the detection comprises the steps of: (a) incubating the sample with at least one antibiotic to allow for enrichment of bacteria that are resistant to the antibiotic, (b) incubating the enriched samples with bacteriophage that infects the bacterium of interest, wherein the bacteriophage comprises an indicator gene such that expression of the indicator gene during bacteriophage replication following infection of the bacterium of interest results in production of a soluble indicator protein product; and (c) detecting the indicator protein product, wherein positive detection of the indicator protein product indicates that the antibiotic-resistant bacterium of interest is present in the sample; (iii) initiating a treatment for the subject; (iv) providing a second biological sample of the same type as the first biological sample obtained from the subject; (v) detecting the specific microorganism or category of microorganisms in the second biological sample using the indicator phage; and (vi) determining a decrease, increase, or steady level of the specific microorganism or category of microorganisms in the subject based on the amounts detected in the first and second biological samples.

### BRIEF DESCRIPTION OF THE FIGURES

The present invention may be better understood by referring to the following nonlimiting figures.

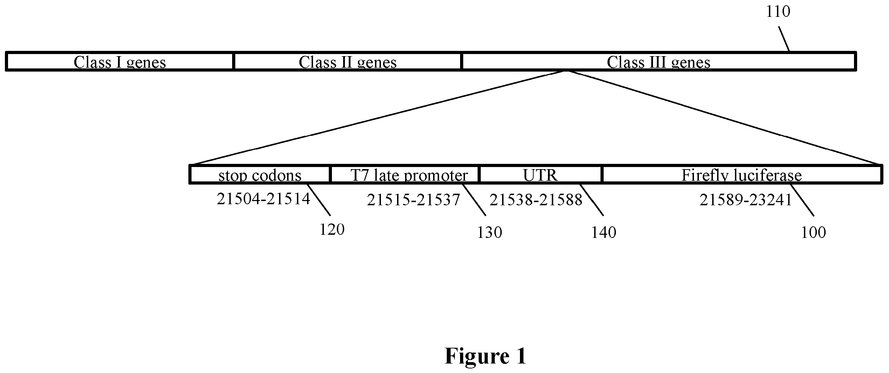

**Figure 1** depicts an indicator phage construct according to an embodiment of the invention illustrating insertion of a genetic construct comprising Firefly luciferase gene and a T7 late promoter inserted into the late (class **III)** region of a bacteriophage. Also depicted is a sequence comprising stop codons in all three reading frames to prevent read-through and an untranslated region (UTR).

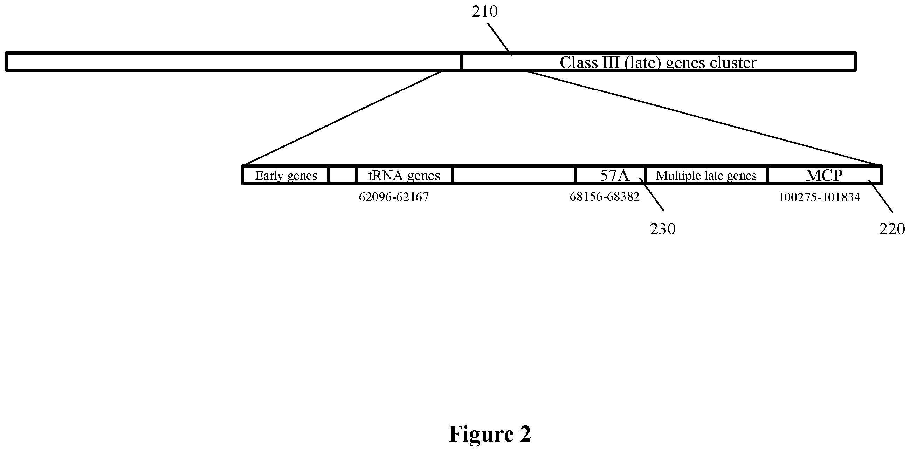

**Figure 2** shows the genome of bacteriophage SEA1, a myovirus (related to T4 bacteriophage) which was obtained from the lab of Francisco Diez-Gonzalez and shares ~95% homology with myovirus *Salmonella* Phage S16. Gene 57A chaperone for long tail fiber formation is at the periphery of the late gene region, consisting of structural genes, which code for virion proteins. As these virion proteins are expressed at a very high level, any genes inserted into this region can be expected to have similar expression levels, as long as late gene promoters and/or other similar control elements are used.

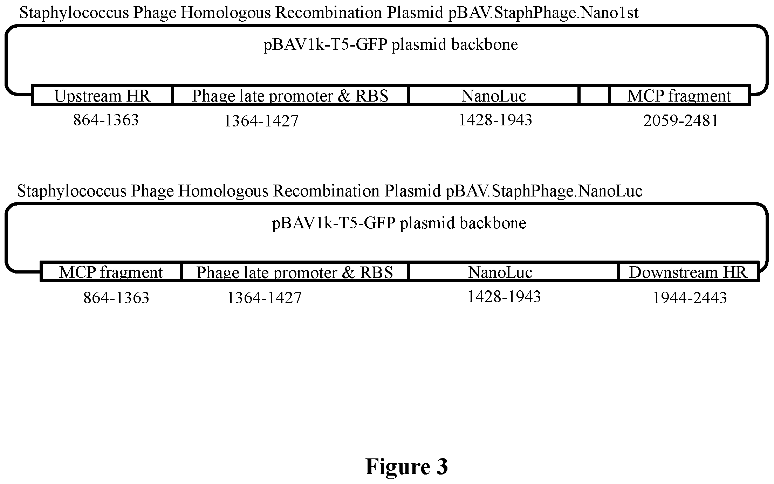

**Figure 3** shows two homologous recombination plasmid constructs carrying luciferase genes for 2 different phages with approximately 500bp of matching phage sequence upstream and downstream of the insertion site to promote homologous recombination. NANOLUC^{®} luciferase is inserted into a pBAV1k-T5-GFP plasmid backbone with an upstream untranslated region containing a phage late gene promoter and Ribosomal Entry Site. The *S. aureus* phage recombination plasmid was constructed to insert NANOLUC^{®} within the late gene region, but at a distance from the Major Capsid Protein (MCP) due to stability issues.

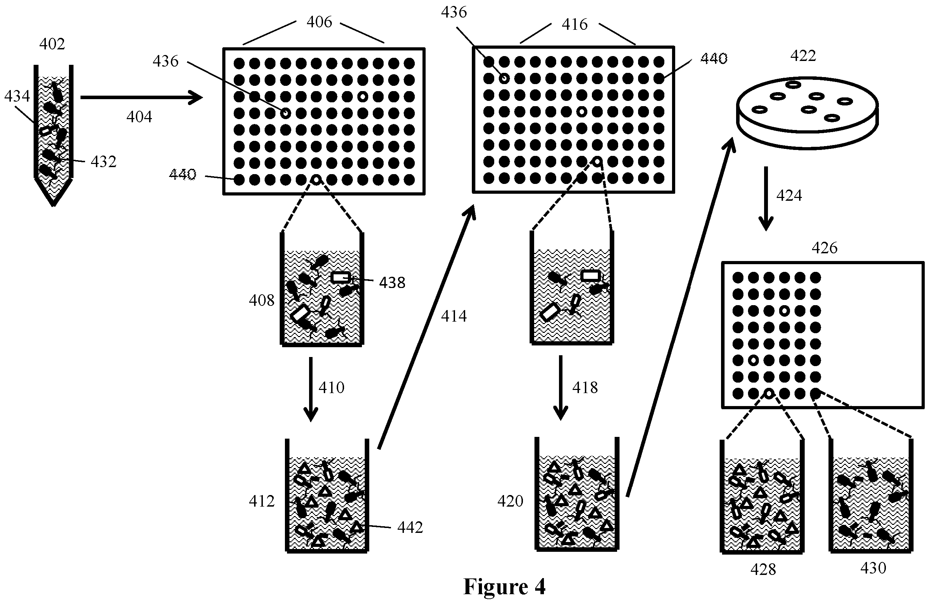

**Figure 4** depicts the isolation of recombinant phage from modifications of *S. aureus* bacteriophage using the plasmid constructs such as those shown in **Figure 3** using a series of sequential infection and dilution steps to identify recombinant phage that express an indicator gene.

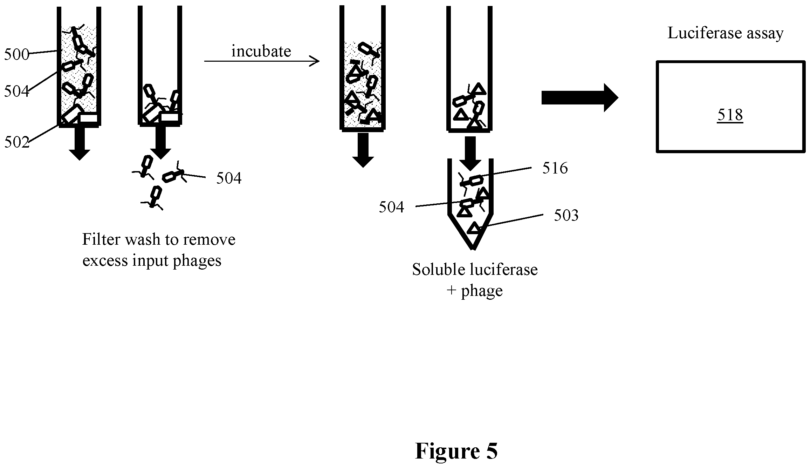

**Figure 5** depicts the use of indicator phage encoding a soluble luciferase to detect bacterial cells via detection of luciferase generated from replication of progeny phage during infection of the bacterial cells, according to an embodiment of the invention.

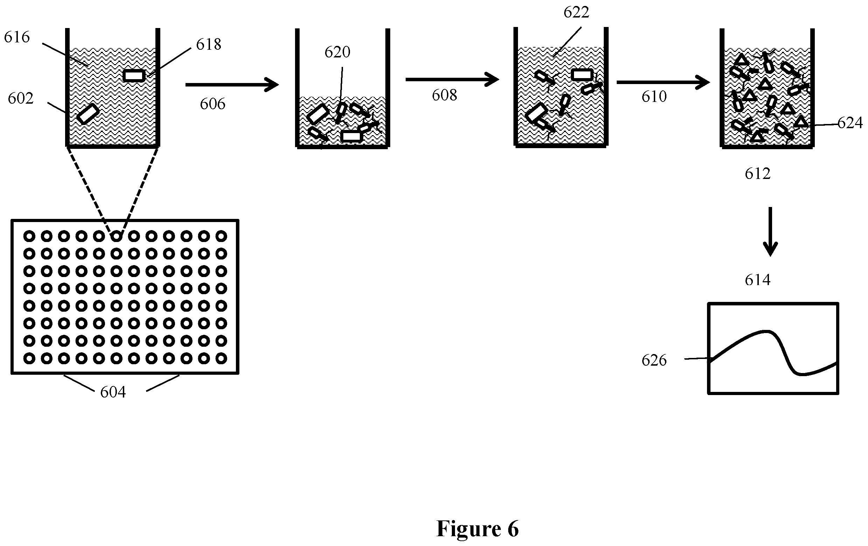

**Figure 6** depicts a filter plate assay for detecting bacteria of interest using a modified bacteriophage according to an embodiment of the invention where bacteria and recombinant phage are incubated on filter plates and after generation of progeny bacteriophage the indicator protein is detected directly without removal of the incubation medium.

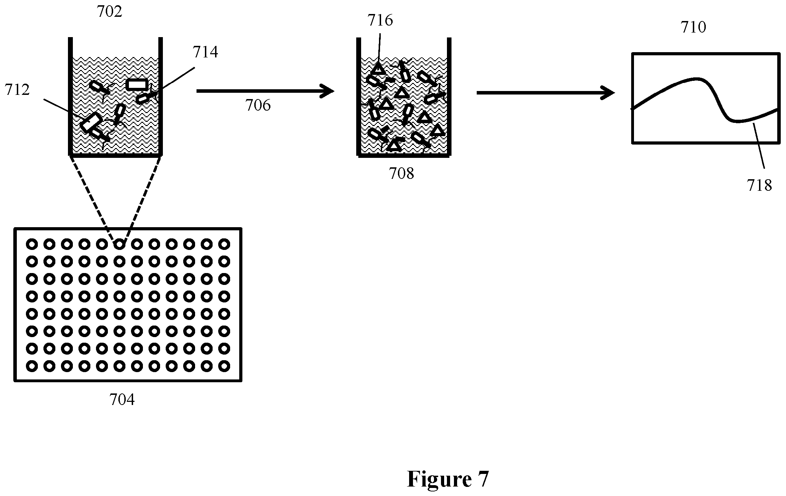

**Figure 7** depicts a "No Concentration Assay" for detecting a bacterium of interest using a modified bacteriophage according to an embodiment of the invention.

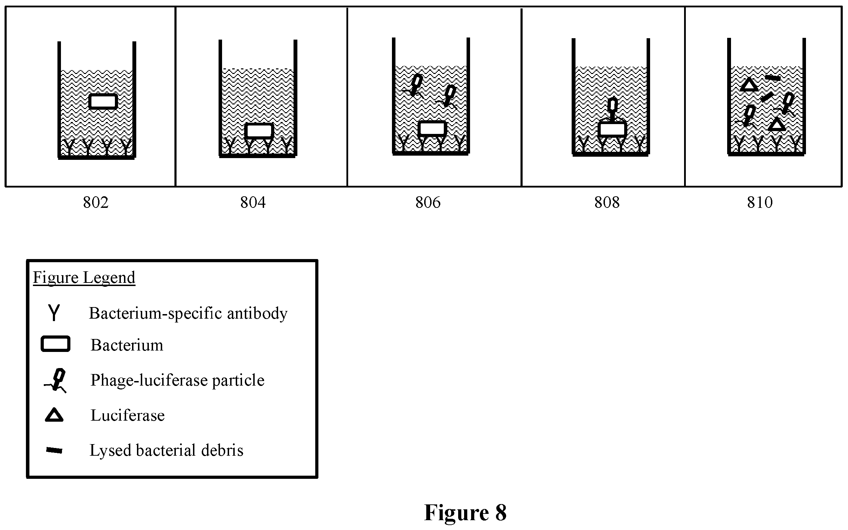

**Figure 8** depicts a Hybrid Immuno-Phage (HIP) Assay for detecting a bacterium of interest using a modified bacteriophage according to an embodiment of the invention wherein antibodies to the microorganism of interest are used to capture the microorganism on the surface of the assay well prior to incubation with a recombinant infectious agent having an indicator gene.

### DETAILED DESCRIPTION OF THE INVENTION

Disclosed herein are methods that demonstrate surprising sensitivity for detection of a microorganism of interest in test samples (e.g., biological, food, water, and clinical samples). Detection can be achieved in a shorter timeframe than was previously thought possible using genetically modified infectious agents in assays performed without culturing for enrichment, or in some embodiments with minimal incubation times during which microorganisms could potentially multiply. Also surprising is the success of using a potentially high multiplicity of infection (MOI), or high concentrations of plaque forming units (PFU), for incubation with a test sample. Such high phage concentrations (PFU/mL) were previously purported to be detrimental in bacterium detection assays, as they were purported to cause "lysis from without." However, a high concentration of phage can facilitate finding, binding, and infecting a low number of target cells.

The methods of the invention comprise infectious agents for use in detection of such microorganisms. The invention comprises a composition comprising a recombinant bacteriophage having an indicator gene inserted into a late gene region of the bacteriophage. The expression of the indicator gene during bacteriophage replication following infection of a host bacterium results in production of a soluble indicator protein product. In certain embodiments, the indicator gene may be inserted into a late gene (i.e., class III) region of the bacteriophage. The bacteriophage can be derived from T7, T4, T4-like, Phage K, MP131, MP115, MP112, MP506, MP87, Rambo, SAPJV1, or *Staphylococcus-specific* bacteriophage, or another wild-type or engineered bacteriophage.

The invention comprises a method for detecting a microorganism of interest. The method uses an infectious agent for detection of the microorganism of interest. The microorganism of interest is a bacterium and the infectious agent is a bacteriophage. Thus, the method comprises detection of a bacterium of interest in a sample by incubating the sample with a recombinant bacteriophage that infects the bacterium of interest. The recombinant bacteriophage comprises an indicator gene. The indicator gene may, in certain embodiments, be inserted into a late gene region of the bacteriophage such that expression of the indicator gene during bacteriophage replication following infection of host bacteria results in production of an indicator protein product. The method comprises detecting the indicator protein product, wherein positive detection of the indicator protein product indicates that the bacterium of interest is present in the sample. The indicator protein is soluble.

Thus, some embodiments of the present invention solve a need by using bacteriophage-based methods for amplifying a detectable signal indicating the presence of bacteria. In certain embodiments as little as a single bacterium is detected. The principles applied herein can be applied to the detection of a variety of microorganisms. Because of numerous binding sites for an infectious agent on the surface of a microorganism, the capacity to produce one hundred or more agent progeny during infection, and the potential for high level expression of an encoded indicator moiety, the infectious agent or an indicator moiety can be more readily detectable than the microorganism itself. In this way, embodiments of the present invention can achieve tremendous signal amplification from even a single infected cell.

Aspects of the present invention utilize the high specificity of binding agents that can bind to particular microorganisms, such as the binding component of infectious agents, as a means to detect and/or quantify the specific microorganism in a sample. The present invention utilizes the high specificity of infectious agents such as bacteriophage.

Detection is achieved through an indicator moiety associated with the binding agent specific for the microorganism of interest. The infectious agent comprises a gene encoding a soluble indicator protein product. In some embodiments the indicator may be encoded by the infectious agent, such as a bacteriophage, and the bacteriophage is designated an indicator phage.

The invention disclosed and described herein utilize the discovery that a single microorganism is capable of binding specific recognition agents, such as phage. Following infection and replication of the phage, progeny phage may be detected via an indicator moiety expressed during phage replication. This principle allows amplification of indicator signal from one or a few cells based on specific recognition of microorganism surface receptors. For example, by exposing even a single cell of a bacterium to a plurality of phage, thereafter allowing amplification of the phage and high-level expression of an encoded indicator gene product during replication, the indicator signal is amplified such that the single bacterium is detectable.

Embodiments of the methods of the invention can be applied to detection and quantification of a variety of microorganisms (e.g., bacteria, fungi, yeast) in a variety of circumstances, including but not limited to detection of pathogens from food, water, clinical and commercial samples. In some embodiments, clinical samples can be analyzed for the presence of microorganisms. The methods of the present invention provide high detection sensitivity and specificity rapidly and without the need for traditional biological enrichment (e.g., culturing for enrichment), which is a surprising aspect as all available methods require culturing. In some embodiments detection is possible within a single replication cycle of the bacteriophage, which is unexpected.

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. Known methods and techniques are generally performed according to conventional methods well known in the art and as described in various general and more specific references that are discussed throughout the present specification unless otherwise indicated. Enzymatic reactions and purification techniques are performed according to manufacturer's specifications, as commonly accomplished in the art or as described herein. The nomenclatures used in connection with the laboratory procedures and techniques described herein are those well-known and commonly used in the art.

The following terms, unless otherwise indicated, shall be understood to have the following meanings:
As used herein, the terms "a", "an", and "the" can refer to one or more unless specifically noted otherwise.

The use of the term "or" is used to mean "and/or" unless explicitly indicated to refer to alternatives only or the alternatives are mutually exclusive, although the disclosure supports a definition that refers to only alternatives and "and/or." As used herein "another" can mean at least a second or more.

Throughout this application, the term "about" is used to indicate that a value includes the inherent variation of error for the device, the method being employed to determine the value, or the variation that exists among samples.

The term "solid support" or "support" means a structure that provides a substrate and/or surface onto which biomolecules may be bound. For example, a solid support may be an assay well (i.e., such as a microtiter plate or multi-well plate), or the solid support may be a location on a filter, an array, or a mobile support, such as a bead or a membrane (e.g., a filter plate or lateral flow strip).

The term "binding agent" refers to a molecule that can specifically and selectively bind to a second (i.e., different) molecule of interest. The interaction may be non-covalent, for example, as a result of hydrogen bonding, van der Waals interactions, or electrostatic or hydrophobic interactions, or it may be covalent. The term "soluble binding agent" refers to a binding agent that is not associated with (i.e., covalently or non-covalently bound) to a solid support.

As used herein, an "analyte" refers to a molecule, compound or cell that is being measured. The analyte of interest may, in certain embodiments, interact with a binding agent. As described herein, the term "analyte" may refer to a protein or peptide of interest. An analyte may be an agonist, an antagonist, or a modulator. Or, an analyte may not have a biological effect. Analytes may include small molecules, sugars, oligosaccharides, lipids, peptides, peptidomimetics, organic compounds and the like.

The term "detectable moiety" or "detectable biomolecule" or "reporter" or "indicator" or "indicator moiety" refers to a molecule that can be measured in a quantitative assay. For example, an indicator moiety may comprise an enzyme that may be used to convert a substrate to a product that can be measured. An indicator moiety may be an enzyme that catalyzes a reaction that generates bioluminescent emissions (e.g., luciferase). Or, an indicator moiety may be a radioisotope that can be quantified. Or, an indicator moiety may be a fluorophore. Or, other detectable molecules may be used.

As used herein, "bacteriophage" or "phage" includes one or more of a plurality of bacterial viruses. In this disclosure, the terms "bacteriophage" and "phage" include viruses such as mycobacteriophage (such as for TB and paraTB), mycophage (such as for fungi), mycoplasma phage, and any other term that refers to a virus that can invade living bacteria, fungi, mycoplasma, protozoa, yeasts, and other microscopic living organisms and uses them to replicate itself. Here, "microscopic" means that the largest dimension is one millimeter or less. Bacteriophages are viruses that have evolved in nature to use bacteria as a means of replicating themselves. A phage does this by attaching itself to a bacterium and injecting its DNA (or RNA) into that bacterium, and inducing it to replicate the phage hundreds or even thousands of times. This is referred to as phage amplification.

As used herein, "late gene region" refers to a region of a viral genome that is transcribed late in the viral life cycle. The late gene region typically includes the most abundantly expressed genes (e.g., structural proteins assembled into the bacteriophage particle). Late genes are synonymous with class III genes and include genes with structure and assembly functions. For example, the late genes (synonymous with class III,) are transcribed in phage T7, e.g., from 8 minutes after infection until lysis, class I (e.g., RNA polymerase) is early from 4-8 minutes, and class II from 6-15 minutes, so there is overlap in timing of II and III. A late promoter is one that is naturally located and active in such a late gene region.

As used herein, "culturing for enrichment" refers to traditional culturing, such as incubation in media favorable to propagation of microorganisms, and should not be confused with other possible uses of the word "enrichment," such as enrichment by removing the liquid component of a sample to concentrate the microorganism contained therein, or other forms of enrichment that do not include traditional facilitation of microorganism propagation. Culturing for enrichment for very short periods of time may be employed in some embodiments of methods described herein, but is not necessary and is for a much shorter period of time than traditional culturing for enrichment, if it is used at all.

As used herein "recombinant" refers to genetic (i.e., nucleic acid) modifications as usually performed in a laboratory to bring together genetic material that would not otherwise be found. This term is used interchangeably with the term "modified" herein.

As used herein "RLU" refers to relative light units as measured by a luminometer (e.g., GLOMAX^{®} 96) or similar instrument that detects light. For example, the detection of the reaction between luciferase and appropriate substrate (e.g., NANOLUC^{®} with NANO-GLO^{®}) is often reported in RLU detected.

As used herein "time to results" refers to the total amount of time from beginning of sample incubation to generated result. Time to results does not include any confirmatory testing time. Data collection can be done at any time after a result has been generated.

### Samples

Each of the embodiments of the methods of the invention can allow for the rapid detection and quantification of microbes in a sample. For example, methods according to the present invention can be performed in a shortened time period with superior results.

Microbes detected by the methods of the present invention include pathogens that are of natural, commercial, medical or veterinary concern. Such pathogens include Gram-negative bacteria, Gram-positive bacteria, and mycoplasmas. Any microbe for which an infectious agent that is specific for the particular microbe has been identified can be detected by the methods of the present invention.

Bacterial cells detectable by the present invention include methicillin-resistant *Staphylococcus aureus* (MRSA).

The sample may be an environmental or food or water sample. Some embodiments may include medical or veterinary samples. Samples may be liquid, solid, or semi-solid. Samples may be swabs of solid surfaces. Samples may include environmental materials, such as the water samples, or the filters from air samples or aerosol samples from cyclone collectors. Samples may be of vegetables, meat, fish, poultry, peanut butter, processed foods, powdered infant formula, powdered milk, teas, starches, eggs, milk, cheese, or other dairy products. Medical or veterinary samples include, but are not limited to, blood, sputum, cerebrospinal fluid, and fecal samples and different types of swabs.

In some embodiments, samples may be used directly in the detection methods of the present invention, without preparation, concentration, or dilution. For example, liquid samples, including but not limited to, milk and juices, may be assayed directly. Samples may be diluted or suspended in solution, which may include, but is not limited to, a buffered solution or a bacterial culture medium. A sample that is a solid or semi-solid may be suspending in a liquid by mincing, mixing or macerating the solid in the liquid. A sample should be maintained within a pH range that promotes bacteriophage attachment to the host bacterial cell. A sample should also contain the appropriate concentrations of divalent and monovalent cations, including but not limited to Na⁺, Mg²⁺, and Ca2⁺. Preferably a sample is maintained at a temperature that maintains the viability of any pathogen cells contained within the sample.

Preferably throughout detection assays, the sample is maintained at a temperature that maintains the viability of any pathogen cell present in the sample. During steps in which bacteriophages are attaching to bacterial cells, it is preferable to maintain the sample at a temperature that facilitates bacteriophage attachment. During steps in which bacteriophages are replicating within an infected bacterial cell or lysing such an infected cell, it is preferable to maintain the sample at a temperature that promotes bacteriophage replication and lysis of the host. Such temperatures are at least about 25 degrees Celsius (C), more preferably no greater than about 45 degrees C, most preferably about 37 degrees C. It is also preferred that the samples be subjected to gentle mixing or shaking during bacteriophage attachment, replication and cell lysis.

Assays may include various appropriate control samples. For example, control samples containing no bacteriophages or control samples containing bacteriophages without bacteria may be assayed as controls for background signal levels.

### Indicator Bacteriophage

As described in more detail herein, the methods of the invention may comprise infectious agents for use in detection of pathogenic microorganisms. The invention comprises a recombinant indicator bacteriophage, wherein the bacteriophage genome is genetically modified to include an indicator or reporter gene. In some embodiments, the invention may include a composition comprising a recombinant bacteriophage having an indicator gene incorporated into the genome of the bacteriophage.

A recombinant indicator bacteriophage can include a indicator gene. In certain embodiments of the infectious agent, the indicator gene does not encode a fusion protein. For example, in certain embodiments, expression of the indicator gene during bacteriophage replication following infection of a host bacterium results in a soluble indicator protein product. In certain embodiments, the indicator gene may be inserted into a late gene region of the bacteriophage. Late genes are generally expressed at higher levels than other phage genes, as they code for structural proteins. The late gene region may be a class III gene region and may include a gene for a major capsid protein.

Some embodiments include designing (and optionally preparing) a sequence for homologous recombination downstream of the major capsid protein gene. Other embodiments include designing (and optionally preparing) a sequence for homologous recombination upstream of the major capsid protein gene. In some embodiments, the sequence comprises a codon-optimized reporter gene preceded by an untranslated region. The untranslated region may include a phage late gene promoter and ribosomal entry site.

In some embodiments, an indicator bacteriophage is derived from T7, T4, T4-like, Phage K, MP131, MP115, MP112, MP506, MP87, Rambo, SAPJV1, or *Staphylococcus-specific* bacteriophage, or another bacteriophage having a genome with at least 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 % homology to T7, T4, T4-like, Phage K, MP131, MP115, MP112, MP506, MP87, Rambo, SAPJV1, or *Staphylococcus-specific* bacteriophage. In some embodiments, the indicator phage is derived from a bacteriophage that is highly specific for a particular pathogenic microorganism. The genetic modifications may avoid deletions of wild-type genes and thus the modified phage may remain more similar to the wild-type infectious agent than many commercially available phage. Environmentally derived bacteriophage may be more specific for bacteria that are found in the environment and as such, genetically distinct from phage available commercially.

Moreover, phage genes thought to be nonessential may have unrecognized function. For example, an apparently nonessential gene may have an important function in elevating burst size such as subtle cutting, fitting, or trimming functions in assembly. Therefore, deleting genes to insert an indicator may be detrimental. Most phages can package a DNA that is a few percent larger than their natural genome. With this consideration, a smaller indicator gene may be a more appropriate choice for modifying a bacteriophage, especially one with a smaller genome. OpLuc and NANOLUC^{®} proteins are only about 20 kDa (approximately 500-600 bp to encode), while FLuc is about 62 kDa (approximately 1,700 bp to encode). For comparison, the genome of T7 is around 40 kbp, while the T4 genome is about 170 kbp, and the genome of *Staphylococcus-*specific bacteriophage is about 157 kbp. Moreover, the reporter gene should not be expressed endogenously by the bacteria (i.e., is not part of the bacterial genome), should generate a high signal to background ratio, and should be readily detectable in a timely manner. Promega's NANOLUC^{®} is a modified Oplophorus gracilirostris (deep sea shrimp) luciferase. In some embodiments, NANOLUC^{®} combined with Promega's NANO-GLO^{®}, an imidazopyrazinone substrate (furimazine), can provide a robust signal with low background.

In some indicator phage embodiments, the indicator gene can be inserted into an untranslated region to avoid disruption of functional genes, leaving wild-type phage genes intact, which may lead to greater fitness when infecting non-laboratory strains of bacteria. Additionally, including stop codons in all three reading frames may help to increase expression by reducing read-through, also known as leaky expression. This strategy may also eliminate the possibility of a fusion protein being made at low levels, which would manifest as background signal (e.g., luciferase) that cannot be separated from the phage.

An indicator gene may express a variety of biomolecules. The indicator gene is a gene that expresses a detectable product or an enzyme that produces a detectable product. For example, in one embodiment the indicator gene encodes a luciferase enzyme. Various types of luciferase may be used. In alternate embodiments, and as described in more detail herein, the luciferase is one of Oplophorus luciferase, Firefly luciferase, Lucia luciferase, Renilla luciferase, or an engineered luciferase. In some embodiments, the luciferase gene is derived from *Oplophorus.* In some embodiments, the indicator gene is a genetically modified luciferase gene, such as NANOLUC^{®}.

Thus, in some embodiments, the present invention comprises a genetically modified bacteriophage comprising a non-bacteriophage indicator gene in the late (class III) gene region. In some embodiments, the non-native indicator gene is under the control of a late promoter. Using a viral late gene promoter insures the reporter gene (e.g., luciferase) is not only expressed at high levels, like viral capsid proteins, but also does not shut down like endogenous bacterial genes or even early viral genes.

In some embodiments, the late promoter is a T7, T4, T4-like, Phage K, MP131, MP115, MP112, MP506, MP87, Rambo, SAPJV1 promoter, or another phage promoter similar to that found in the selected wild-type phage, i.e., without genetic modification. The late gene region may be a class III gene region, and the bacteriophage may be derived from T7, T4, T4-like, Phage K, MP131, MP115, MP112, MP506, MP87, Rambo, SAPJV1, *Staphylococcus-, or S. aureus-specific* bacteriophage, or another natural bacteriophage having a genome with at least 70, 75, 80, 85, 90 or 95% homology to T7, T4, T4-like, Phage K, MP131, MP115, MP112, MP506, MP87, Rambo, SAPJV1, *Staphylococcus-,* or *S. aureus-specific* bacteriophage.

Genetic modifications to infectious agents may include insertions, deletions, or substitutions of a small fragment of nucleic acid, a substantial part of a gene, or an entire gene. In some embodiments, inserted or substituted nucleic acids comprise non-native sequences. A non-native indicator gene may be inserted into a bacteriophage genome such that it is under the control of a bacteriophage promoter. In some embodiments, the non-native indicator gene is not part of a fusion protein. That is, in some embodiments, a genetic modification may be configured such that the indicator protein product does not comprise polypeptides of the wild-type bacteriophage. In some embodiments, the indicator protein product is soluble. In some embodiments, the invention comprises a method for detecting a bacterium of interest comprising the step of incubating a test sample with such a recombinant bacteriophage.

Expression of the indicator gene in progeny bacteriophage following infection of host bacteria results in a free, soluble protein product. In some embodiments, the non-native indicator gene is not contiguous with a gene encoding a structural phage protein and therefore does not yield a fusion protein. Unlike systems that employ a fusion of a detection moiety to the capsid protein (i.e., a fusion protein), the present invention expresses a soluble indicator (e.g., soluble luciferase). In some embodiments, the indicator is ideally free of the bacteriophage structure. That is, the indicator is not attached to the phage structure. As such, the gene for the indicator is not fused with other genes in the recombinant phage genome. This may greatly increase the sensitivity of the assay (down to a single bacterium), and simplifies the assay, allowing the assay to be completed in less than an hour for some embodiments, as opposed to several hours due to additional purification steps required with constructs that produce detectable fusion proteins. Further, fusion proteins may be less active than soluble proteins due, e.g., to protein folding constraints that may alter the conformation of the enzyme active site or access to the substrate.

Moreover, fusion proteins by definition limit the number of the moieties attached to subunits of a protein in the bacteriophage. For example, using a commercially available system designed to serve as a platform for a fusion protein would result in about 415 copies of the fusion moiety, corresponding to the about 415 copies of the gene 10B capsid protein in each T7 bacteriophage particle. Without this constraint, infected bacteria can be expected to express many more copies of the detection moiety (e.g., luciferase) than can fit on the bacteriophage. Additionally, large fusion proteins, such as a capsid-luciferase fusion, may inhibit assembly of the bacteriophage particle, thus yielding fewer bacteriophage progeny. Thus a soluble, non-fusion indicator gene product may be preferable.

In some embodiments, the indicator phage encodes a reporter, such as a detectable enzyme. The indicator gene product may generate light and/or may be detectable by a color change. Various appropriate enzymes are commercially available, such as alkaline phosphatase (AP), horseradish peroxidase (HRP), or luciferase (Luc). In some embodiments, these enzymes may serve as the indicator moiety. In some embodiments, Firefly luciferase is the indicator moiety. In some embodiments, Oplophorus luciferase is the indicator moiety. In some embodiments, NANOLUC^{®} is the indicator moiety. Other engineered luciferases or other enzymes that generate detectable signals may also be appropriate indicator moieties.

In some embodiments, the use of a soluble detection moiety eliminates the need to remove contaminating parental phage from the lysate of the infected sample cells. With a fusion protein system, any bacteriophage used to infect sample cells would have the detection moiety attached, and would be indistinguishable from the daughter bacteriophage also containing the detection moiety. As detection of sample bacteria relies on the detection of a newly created (*de novo* synthesized) detection moiety, using fusion constructs requires additional steps to separate old (parental) moieties from newly created (daughter bacteriophage) moieties. This may be accomplished by washing the infected cells multiple times, prior to the completion of the bacteriophage life cycle, inactivating excess parental phage after infection by physical or chemical means, and/or chemically modifying the parental bacteriophage with a binding moiety (such as biotin), which can then be bound and separated (such as by streptavidin-coated sepharose beads). However, even with all these attempts at removal, parental phage can remain when a high concentration of parental phage is used to assure infection of a low number of sample cells, creating background signal that may obscure detection of signal from infected cell progeny phage.

By contrast, with the soluble detection moiety expressed in some embodiments of the present invention, purification of the parental phage from the final lysate is unnecessary, as the parental phage do not have any detection moiety attached. Thus any detection moiety present after infection must have been created *de novo,* indicating the presence of an infected bacterium or bacteria. To take advantage of this benefit, the production and preparation of parental phage may include purification of the phage from any free detection moiety produced during the production of parental bacteriophage in bacterial culture. Standard bacteriophage purification techniques may be employed to purify some embodiments of phage according to the present invention, such as sucrose density gradient centrifugation, cesium chloride isopycnic density gradient centrifugation, HPLC, size exclusion chromatography, and dialysis or derived technologies (such as Amicon brand concentrators - Millipore, Inc.). Cesium chloride isopycnic ultracentrifugation can be employed as part of the preparation of recombinant phage of the invention, to separate parental phage particles from contaminating luciferase protein produced upon propagation of the phage in the bacterial host. In this way, the parental recombinant bacteriophage of the invention is substantially free of any luciferase generated during production in the bacteria. Removal of residual luciferase present in the phage stock can substantially reduce background signal observed when the recombinant bacteriophage are incubated with a test sample.

In some embodiments of modified bacteriophage, the late promoter (class III promoter, e.g., from T7, T4, or ViI) has high affinity for RNA polymerase of the same bacteriophage that transcribes genes for structural proteins assembled into the bacteriophage particle. These proteins are the most abundant proteins made by the phage, as each bacteriophage particle comprises dozens or hundreds of copies of these molecules. The use of a viral late promoter can ensure optimally high level of expression of the luciferase detection moiety. The use of a late viral promoter derived from, specific to, or active under the original wild-type bacteriophage the indicator phage is derived from (e.g., a T4, T7, or ViI late promoter with a T4-, T7-, or ViI- based system) can further ensure optimal expression of the detection moiety. The use of a standard bacterial (non-viral/non-bacteriophage) promoter may in some cases be detrimental to expression, as these promoters are often down-regulated during bacteriophage infection (in order for the bacteriophage to prioritize the bacterial resources for phage protein production). Thus, in some embodiments, the phage is preferably engineered to encode and express at high level a soluble (free) indicator moiety, using a placement in the genome that does not limit expression to the number of subunits of a phage structural component.

### Methods of Preparing Indicator Bacteriophage

Methods for making indicator bacteriophage begin with selection of a wild-type bacteriophage for genetic modification. Some bacteriophage are highly specific for a target bacterium. This presents an opportunity for highly specific detection.

Infectious agents (e.g., bacteriophage) specifically recognize surface receptors of particular microorganisms and thus specifically infect those microorganisms. As such, these infectious agents may be appropriate binding agents for targeting a microorganism of interest.

A variety of infectious agents may be used. Bacteriophages, phages, mycobacteriophages (such as for TB and paraTB), mycophages (such as for fungi), mycoplasma phages, and any other virus that can invade living bacteria, fungi, mycoplasma, protozoa, yeasts, and other microscopic living organisms can be employed to target a microorganism of interest. For example, where the microorganism of interest is a bacterium, the infectious agent may comprise a bacteriophage. For example, well-studied phages of *E. coli* include T1, T2, T3, T4, T5, T7, and lambda; other *E*. *coli* phages available in the ATCC collection, for example, include phiX174, S13, Ox6, MS2, phiV1, fd, PR772, and ZIK1. As discussed herein, the bacteriophage may replicate inside of the bacteria to generate hundreds of progeny phage. Detection of the product of an indicator gene inserted into the bacteriophage genome can be used as a measure of the bacteria in the sample.

The invention utilizes the specificity of binding and high-level genetic expression capacity of recombinant bacteriophage for rapid and sensitive targeting to infect and facilitate detection of a bacterium of interest. In some embodiments, methicillin-resistant *Staphylococcus-* or *S*. *aureus-*specific bacteriophage is genetically modified to include a reporter gene. In some embodiments the late gene region of a bacteriophage is genetically modified to include a reporter gene. In some embodiments, a reporter gene is positioned downstream of the major capsid gene. In other embodiments, a reporter gene is positioned upstream of the major capsid gene.

Methods for preparing a recombinant indicator bacteriophage include selecting a wild-type bacteriophage that specifically infects a target pathogenic bacterium; preparing a homologous recombination plasmid/vector that comprises an indicator gene; transforming the homologous recombination plasmid/vector into target pathogenic bacteria; infecting the transformed target pathogenic bacteria with the selected wild-type bacteriophage, thereby allowing homologous recombination to occur between the plasmid/vector and the bacteriophage genome; and isolating a particular clone of recombinant bacteriophage.

Various methods for designing and preparing a homologous recombination plasmid are known. Various methods for transforming bacteria with a plasmid are known, including heat-shock, F pilus mediated bacterial conjugation, electroporation, and other methods. Various methods for isolating a particular clone following homologous recombination are also known. Some method embodiments described herein utilize particular strategies.

The bacteriophage utilized in the methods of the invention are prepared using the following steps of selecting a wild-type bacteriophage that specifically infects a target pathogenic bacterium; determining the natural sequence in the late region of the genome of the selected bacteriophage; annotating the genome and identifying the major capsid protein gene of the selected bacteriophage; designing a sequence for homologous recombination adjacent to the major capsid protein gene, wherein the sequence comprises a codon-optimized reporter gene; incorporating the sequence designed for homologous recombination into a plasmid/vector; transforming the plasmid/vector into target pathogenic bacteria; selecting for the transformed bacteria; infecting the transformed bacteria with the selected wild-type bacteriophage, thereby allowing homologous recombination to occur between the plasmid and the bacteriophage genome; determining the titer of the resulting recombinant bacteriophage lysate; and performing a limiting dilution assay to enrich and isolate the recombinant bacteriophage. Some embodiments comprise further repeating the limiting dilution and titer steps, following the first limiting dilution assay, as needed until the recombinant bacteriophage represent a detectable fraction of the mixture. For example, in some embodiments the limiting dilution and titer steps can be repeated until at least 1/30 of the bacteriophage in the mixture are recombinant before isolating a particular clone of recombinant bacteriophage. A ratio of 1:30 recombinant: wild-type is expected, in some embodiments, to yield an average of 3.2 transducing units (TU) per 96 plaques (e.g., in a 96-well plate). The initial ratio of recombinant to wild-type phage may be determined by performing limiting dilution assays based on the TCID50 (tissue culture infectious dose 50%) as previously described in U.S. App. No. 15/409,258. By Poisson distribution, a 1:30 ratio generates a 96% chance of observing at least one TU somewhere in the 96 wells.

**Figure 1** depicts a schematic representation of the genomic structure of a recombinant bacteriophage of the invention, Indicator Phage T7SELECT^{®}415-Luc. For the embodiment depicted in **Figure 1****,** the detection moiety is encoded by a Firefly luciferase gene 100 inserted within the late (class III) gene region **110,** which is expressed late in the viral life cycle. Late genes are generally expressed at higher levels than other phage genes, as they code for structural proteins. Thus, in the embodiment of the recombinant phage depicted by **Figure 1****,** the indicator gene (i.e., Firefly luciferase) is inserted into the late gene region, just after gene 10B (major capsid protein), and is a construct comprising the Firefly luciferase gene **100.** The construct depicted in **Figure 1** was designed to include stop codons **120** in all 3 reading frames to ensure luciferase is not incorporated into the gene 10B product. Also as depicted by **Figure 1****,** the construct may comprise the consensus T7 late promoter **130** to drive transcription and expression of the luciferase gene. The construct may also comprise a composite untranslated region synthesized from several T7 UTRs **140.** This construct ensures soluble Firefly luciferase is produced such that expression is not limited to the number of capsid proteins inherent in the phage display system.

As noted herein, in certain embodiments, it may be preferred to utilize infectious agents that have been isolated from the environment for production of the infectious agents of the invention. In this way, infectious agents that are specific to naturally derived microorganisms may be generated.

For example, **Figure 2** shows the genome of bacteriophage SEA1, a natural phage having about 95 % sequence homology to a T4 related myovirus bacteriophage S16. SEA1 bacteriophage was obtained from the lab of Francisco Martinez, and whole genome sequencing performed using the Illumina MiSeq with de novo sequence assembly. As discussed in the Examples, the Major Capsid Protein **220** and various other structural genes are within the late gene region **210,** consisting of structural genes, which code for virion proteins. Gene 57A **230,** coding for chaperone for long tail fiber formation lies at the border of the late gene region. As these virion proteins are expressed at a very high level, any genes inserted into this region can be expected to have similar expression levels, as long as late gene promoters and/or other similar control elements are used.

There are numerous known methods and commercial products for preparing plasmids. For example PCR, site-directed mutagenesis, restriction digestion, ligation, cloning, and other techniques may be used in combination to prepare plasmids. Synthetic plasmids can also be ordered commercially (e.g., GeneWiz). Cosmids can also be employed, or the CRISPR/CAS9 system could be used to selectively edit a bacteriophage genome. Methods of preparing a recombinant indicator bacteriophage include designing a plasmid that can readily recombine with the wild-type bacteriophage genome to generate recombinant genomes. Designing a plasmid, includes addition of a codon-optimized reporter gene, such as a luciferase gene. Further including addition of elements into the upstream untranslated region. For example, in designing a plasmid to recombine with the *Staphylococcus- or S. aureus-*specific bacteriophage genome, an upstream untranslated region can be added between the sequence encoding the C-terminus of the gp23 / Major Capsid Protein and the start codon of the NANOLUC^{®} reporter gene. The untranslated region can include a promoter, such as a T7, T4, T4-like, Phage K, MP131, MP115, MP112, MP506, MP87, Rambo, SAPJV1 promoter. The untranslated region can also include a Ribosomal Entry / Binding Site (RBS), also known as a "Shine-Dalgarno Sequence" with bacterial systems. Either or both of these elements, or other untranslated elements, can be embedded within a short upstream untranslated region made of random sequences comprising about the same GC content as rest of the phage genome. The random region should not include an ATG sequence, as that will act as a start codon.

The compositions of the invention comprise various infectious agents and indicator genes. For example, **Figure 3** shows two homologous recombination plasmid constructs carrying luciferase genes for 2 different phages with approximately 500bp of matching phage sequence upstream and downstream of the insertion site to promote homologous recombination. NANOLUC^{®} luciferase is inserted into a pBAV1k-T5-GFP plasmid backbone with an upstream untranslated region containing a phage late gene promoter and Ribosomal Entry Site. The *S*. *aureus* phage recombination plasmid was constructed to insert NANOLUC^{®} within the late gene region, but at a distance from the Major Capsid Protein (MCP) due to stability issues.

The Major Capsid Protein fragment 416-915 is a part of a structural gene that encodes a virion protein. As these virion proteins are expressed at a very high level, any genes inserted into this region can be expected to have similar expression levels, as long as late gene promoters and/or other similar control elements are used.

In some embodiments, indicator phage according to the invention comprise *Staphylococcus-specific* bacteriophage genetically engineered to comprise a reporter gene such as a luciferase gene. For example, an indicator phage can *Staphylococcus-specific* bacteriophage wherein the genome comprises the sequence of the NANOLUC^{®} gene. A recombinant *Staphylococcus-specific* NanoLuc bacteriophage genome may further comprise a promoter such as a T7, T4, T4-like, Phage K, MP131, MP115, MP112, MP506, MP87, Rambo, SAPJV1, *Staphylococcus-specific,* ViI, or another late promoter. In some embodiments, the *Staphylococcus-specific* bacteriophage is a *Staphylococcus* aureus-specific bacteriophage.

Thus, in the embodiment of the recombinant phage generated as a result of the recombination, the indicator gene (i.e., NANOLUC^{®}) is inserted into the late gene region, just downstream of the gene encoding the major capsid protein, and thus creates recombinant bacteriophage genomes comprising the NANOLUC^{®} gene. The construct may additionally comprise the consensus promoter of T7, T4, T4-like, Phage K, MP131, MP115, MP112, MP506, MP87, Rambo, SAPJV1, *Staphylococcus-specific* bacteriophage, ViI, or another late promoter or another suitable promoter to drive transcription and expression of the luciferase gene. The construct may also comprise a composite untranslated region synthesized from several UTRs. This construct ensures soluble luciferase is produced such that expression is not limited to the number of capsid proteins inherent in the phage display system.

**Figure 4** depicts the isolation of recombinant phage from the mixture of wild-type and recombinant bacteriophage resulting from the homologous recombination.

In the first step **402,** *S*. *aureus* bacteria transformed with the homologous recombination plasmid are infected with *S. aureus* bacteriophage Phage K, resulting in progeny phage with a mixture of parental and recombinant phage with a ratio of approximately 186 wild-type to 1 recombinant phage **434.** The resulting recombinant phage mix is diluted **404** into 96-well plates **406** to give an average of 5 recombinant transducing units (TU) per plate (9.3 PFU/well). The 96-well plate is assayed for luciferase activity to identify wells **436** containing recombinant phage as compared to wells **440** containing wild-type bacteriophage. Bacteria **438** are added **408;** for example, each well may contain about 50 µL of a turbid *S, aureus* culture. This allows the phage to replicate and produce the luciferase enzyme **442.** After 2 hours of incubation at 37°C shown in **410,** wells may be screened for the presence of luciferase **442.** Any positive wells are likely to have been inoculated with a single recombinant phage, and at this stage the mixture may contain a ratio of approximately 9.3 wild-type phage: 1 recombinant, an enrichment over the original 186:1 ratio. In one embodiment, one of the 5 wells found to contain soluble luciferase via luciferase assay, contained phage at an approximate ratio of 2.4 total: 1 recombinant. If necessary (i.e., if the ratio of recombinant: total is lower than 1:30), progeny from this enriched culture **412** may be subjected to additional limiting dilution assay(s) **414** to increase the ratio and determine the actual concentration of recombinant phage transducing units. For example, if the ratio was 1:384 recombinants:PFU, about 5 recombinant TU along with 1920 contaminating total phage (5 x 384 = 1920) per 96-well plate **416** may be aliquoted **414** from the previous positive well, leading to an approximate inoculation of 20 mostly wild-type phage per well (1920 PFU / 96 wells = 20 PFU/well) of a second dilution assay plate **420.** Any positive luciferase wells are likely to have been inoculated with a single recombinant along with 19 wild-type phage. These wells may be analyzed for presence of luciferase **442.**

After addition of bacteria and incubation (e.g., for 2 hours at 37°C) **418,** soluble luciferase and phage are present at approximately 20 total: 1 recombinant **420.** This ratio may be verified by TU50 titration for recombinants and plaque assay for total PFU. Finally, a plaque assay may be performed **422** to screen for recombinants that express luciferase **446.** A small number of individual (e.g., n=48) plaques may be individually picked and screened in a third multiwell plate **426** for luciferase activity **436.** In an embodiment, this approach should insure that enough plaques be screened so about 3 recombinants would be in the mix of plaques being screened based on the known ratio of recombinants to total phage. One plaque may be removed from the plate to each well of a 96-well plate **424** and a luciferase assay performed **426** to determine which wells contained phage exhibiting luciferase activity **442.** Wells **428** demonstrating luciferase activity represent pure recombinant phage **434,** while wells without luciferase activity **430** represent pure wild-type phage **432.**

Individual plaques may then be suspended in buffer (e.g., 100 µL TMS) or media, and an aliquot (e.g., about 5 µL) added to a well containing a turbid *S. aureus* culture, and assayed after incubation (e.g., about 45 minutes to 1 hour at 37°C). Positive wells are expected to contain a pure culture of recombinant phage. Certain embodiments can include additional rounds of plaque purification.

Thus, as illustrated by **Figure 4****,** recombinant phage generated by homologous recombination of a plasmid designed for recombination with the wild-type phage genome can be isolated from a mixture comprising only 0.005% of total phage genomes. Following isolation, large scale production may be performed to obtain high titer recombinant indicator phage stocks appropriate for use in the *S. aureus* detection assay. Furthermore, cesium chloride isopycnic density gradient centrifugation may be used to separate phage particles from contaminating luciferase protein to reduce background.

### Methods of Using Infectious Agents for Detecting Microorganisms

As noted herein, in certain embodiments, the invention may comprise methods of using infectious particles for detecting microorganisms. The methods of the invention may be embodied in a variety of ways.

The invention comprises a method for detecting a bacterium of interest in a sample comprising the steps of: (a) incubating the sample with at least one antibiotic to allow for enrichment of bacteria that are resistant to the antibiotic, (b) incubating the enriched sample with bacteriophage that infects the bacterium of interest, wherein the bacteriophage comprises an indicator gene such that expression of the indicator gene during bacteriophage replication following infection of the bacterium of interest results in production of a soluble indicator protein product; and (c) detecting the indicator protein product, wherein positive detection of the indicator protein product indicates that the bacterium of interest is present in the sample.

The invention comprises a method for detecting an antibiotic-resistant bacteria of interest in a sample comprising the steps of: (a) incubating the sample with at least one antibiotic to allow for enrichment of bacteria that are resistant to the antibiotic, (b) incubating the enriched samples with bacteriophage that infects the bacterium of interest, wherein the bacteriophage comprises an indicator gene such that expression of the indicator gene during bacteriophage replication following infection of the bacterium of interest results in production of a soluble indicator protein product; and (c) detecting the indicator protein product, wherein positive detection of the indicator protein product indicates that the antibiotic-resistant bacterium of interest is present in the sample.

In certain embodiments, the assay may be performed to utilize a general concept that can be modified to accommodate different sample types or sizes and assay formats. Embodiments employing recombinant bacteriophage of the invention (i.e., indicator bacteriophage) may allow rapid detection of specific bacterial strains, with total assay times under 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12, 12.5, 13.0, 13.5, 14.0, 14.5, 15.0, 15.5, 16.0, 16.5, 17.0, 17.5, 18.0, 18.5, 19.0, 19.5, or 20.0 hours, depending on the sample type, sample size, and assay format. For example, the amount of time required may be somewhat shorter or longer depending on the strain of bacteriophage and the strain of bacteria to be detected in the assay, type and size of the sample to be tested, conditions required for viability of the target, complexity of the physical/chemical environment, and the concentration of "endogenous" non-target bacterial contaminants.

**Figure 5** shows a strategy of using indicator phage that produce soluble luciferase according to an embodiment of the invention. In this method, the phage (e.g., T7, T4, T4-like, Phage K, MP131, MP115, MP112, MP506, MP87, Rambo, SAPJV1 phage) may be engineered to express a soluble luciferase during replication of the phage. Expression of luciferase is driven by a viral capsid promoter (e.g., the bacteriophage T7 or T4 late promoter), yielding high expression. Parental phage will be free of luciferase, so the luciferase detected in the assay must come from replication of progeny phage during infection of the bacterial cells. Thus, there is generally no need to separate out the parental phage from the progeny phage.

In these experiments, at least part of the sample **500** comprising the bacteria **502** to be quantified is placed in a spin column filter and centrifuged to remove the LB broth, and an appropriate multiplicity of phage **504** genetically engineered to express soluble luciferase **503** are added. The infected cells may be incubated for a time sufficient for replication of progeny phage and cell lysis to occur (e.g., 30-90 minutes at 37°C). The parental **504** and progeny phage **516** plus free luciferase **503** in the lysate may then be collected, e.g., by centrifugation, and the level of luciferase in the filtrate quantified using a luminometer **518.** Alternatively, a high through-put method may be employed where bacterial samples are applied to a 96-well filter plate, and after all manipulations listed above are performed, may be directly assayed for luciferase in the original 96-well filter plate without a final centrifugation step.

**Figure 6** depicts a filter plate assay for detecting bacteria of interest using a modified bacteriophage according to an embodiment of the invention. Briefly, samples **616** that include a bacterium of interest **618** may be added to wells **602** of a multi-well filter plate 604 and spun **606** to concentrate the samples by removal of liquid from the sample. Genetically modified phage **620** are added to wells and incubated with additional media added for enough time sufficient for adsorption **608** followed by infection of target bacteria and advancement of the phage life cycle **610** (e.g., ~ 45 minutes). Finally, luciferase substrate is added and reacts with any luciferase present **624.** The resulting emission is measured in a luminometer **614** which detects luciferase activity **626.**

In certain embodiments, the assay may be performed without concentrating the bacterium on or near the capture surface. **Figure 7** illustrates a "No Concentration Assay" for detecting a bacterium of interest using a modified bacteriophage according to an embodiment of the invention. Aliquots of indicator phage **714** are distributed to the individual wells **702** of a multi-well plate 704, and then test sample aliquots containing bacteria **712** are added and incubated **706** (e.g., 45 minutes at 37°C) for a period of time sufficient for phage to replicate and generate soluble indicator **716** (e.g., luciferase). The plate wells **708** containing soluble indicator and phage may then be assayed **710** to measure the indicator activity on the plate **718** (e.g., luciferase assay). In this embodiment, the test samples are not concentrated (e.g., by centrifugation) but are simply incubated directly with indicator phage for a period of time and subsequently assayed for luciferase activity.

In some embodiments, the sample may be enriched prior to testing by incubation in conditions that encourage growth. In such embodiments, the enrichment period can be 1, 2, 3, 4, 5, 6, 7, 8, 9, 10, 11, 12, 13, 14, 15, or 16 hours or longer, depending on the sample type and size.

The indicator bacteriophage comprises a detectable indicator moiety, and infection of a single pathogenic cell (e.g., bacterium) can be detected by an amplified signal generated via the indicator moiety. Thus the method comprises detecting an indicator moiety produced during phage replication, wherein detection of the indicator indicates that the bacterium of interest is present in the sample.

The invention may comprise a method for detecting a bacterium of interest in a sample comprising the steps of: (a) incubating the sample with at least one antibiotic to allow for enrichment of bacteria that are resistant to the antibiotic, (b) incubating the enriched sample with a recombinant bacteriophage that infects the bacterium of interest, wherein the recombinant bacteriophage comprises an indicator gene inserted into a late gene region of the bacteriophage such that expression of the indicator gene during bacteriophage replication following infection of host bacteria results in production of a soluble indicator protein product; and detecting the indicator protein product, wherein positive detection of the indicator protein product indicates that the bacterium of interest is present in the sample. In some embodiments, the amount of indicator moiety detected corresponds to the amount of the bacterium of interest present in the sample.

As described in more detail herein, the methods of the invention may utilize a range of concentrations of parental indicator bacteriophage to infect bacteria present in the sample. In some embodiments the indicator bacteriophage are added to the sample at a concentration sufficient to rapidly find, bind, and infect target bacteria that are present in very low numbers in the sample, such as a single cell. In some embodiments, the phage concentration can be sufficient to find, bind, and infect the target bacteria in less than one hour. In other embodiments, these events can occur in less than two hours, or less than three hours, following addition of indicator phage to the sample. For example, in certain embodiments, the bacteriophage concentration for the incubating step is greater than 1 x 10⁵ PFU/mL, greater than 1 x 10⁶ PFU/mL, or greater than 1 x 10⁷ PFU/mL.

In certain embodiments, the recombinant infectious agent may be purified so as to be free of any residual indicator protein that may be generated upon production of the infectious agent stock. Thus, in certain embodiments, the recombinant bacteriophage may be purified using cesium chloride isopycnic density gradient centrifugation prior to incubation with the sample. When the infectious agent is a bacteriophage, this purification may have the added benefit of removing bacteriophage that do not have DNA (i.e., empty phage or "ghosts").

In some embodiments of the methods of the invention, the microorganism may be detected without any isolation or purification of the microorganisms from a sample. For example, in certain embodiments, a sample containing one or a few microorganisms of interest may be applied directly to an assay container such as a spin column, a microtiter well, or a filter and the assay is conducted in that assay container. Various embodiments of such assays are disclosed herein.

Aliquots of a test sample may be distributed directly into wells of a multi-well plate, indicator phage may be added, and after a period of time sufficient for infection, a lysis buffer may be added as well as a substrate for the indicator moiety (e.g., luciferase substrate for a luciferase indicator) and assayed for detection of the indicator signal. Some embodiments of the method can be performed on filter plates. Some embodiments of the method can be performed with or without concentration of the sample before infection with indicator phage.

For example, in many embodiments, multi-well plates are used to conduct the assays. The choice of plates (or any other container in which detecting may be performed) may affect the detecting step. For example, some plates may include a colored or white background, which may affect the detection of light emissions. Generally speaking, white plates have higher sensitivity but also yield a higher background signal. Other colors of plates may generate lower background signal but also have a slightly lower sensitivity. Additionally, one reason for background signal is the leakage of light from one well to another, adjacent well. There are some plates that have white wells but the rest of the plate is black. This allows for a high signal inside the well but prevents well-to-well light leakage and thus may decrease background. Thus the choice of plate or other assay vessel may influence the sensitivity and background signal for the assay.

Methods of the invention may comprise various other steps to increase sensitivity. For example, as discussed in more detail herein, the method may comprise a step for washing the captured and infected bacterium, after adding the bacteriophage but before incubating, to remove excess parental bacteriophage and/or luciferase or other reporter protein contaminating the bacteriophage preparation.

In some embodiments, detection of the microorganism of interest may be completed without the need for culturing the sample as a way to increase the population of the microorganisms. For example, in certain embodiments the total time required for detection is less than 16.0 hours, 15.0 hours, 14.0 hours, 13.0 hours, 12.0 hours, 11.0 hours, 10.0 hours, 9.0 hours, 8.0 hours, 7.0 hours, 6.0 hours, 5.0 hours, 4.0 hours, 3.0 hours, 2.5 hours, 2.0 hours, 1.5 hours, 1.0 hour, 45 minutes, or less than 30 minutes. Minimizing time to result is critical in food and environmental testing for pathogens.

In contrast to assays known in the art, the method of the invention can detect individual microorganisms. Thus, in certain embodiments, the method may detect ≤ 10 cells of the microorganism (i.e., 1, 2, 3, 4, 5, 6, 7, 8, 9 microorganisms) present in a sample. For example, the recombinant bacteriophage is highly specific for *S. aureus,* the *S. aureus* is methicillin-resistant (MRSA). In an embodiment, the recombinant bacteriophage can distinguish *Staphylococcus* in the presence of more than 100 other types of bacteria. In still other embodiments, the recombinant bacteriophage can distinguish *Staphylococcus aureus* in the presence of more than 100 other types of bacteria. In certain embodiments, the recombinant bacteriophage can be used to detect a single bacterium of the specific type in the sample. In certain embodiments, the recombinant bacteriophage detects as few as 2, 3, 4, 5, 6, 7, 8, 9, 10, 15, 20, 30, 40, 50, 60, 70, 80, 90, or 100 of the specific bacteria in the sample.

The invention comprises methods of using recombinant bacteriophage for detecting resistance of microorganisms to an antibiotic or, stated another way, for detecting the efficacy of an antibiotic against a microorganism. Additionally, the methods comprise methods for detecting antibiotic-resistant bacteria in a sample. The methods of the invention may be embodied in a variety of ways.

The method comprises contacting the sample comprising the microorganism with the antibiotic and an infectious agent as described above. A method of determining effective dose of an antibiotic in killing or inhibiting the growth of a microorganism comprising: (a) incubating each of one or more of antibiotic solutions separately with one or more samples comprising the microorganism, wherein the concentrations of the one or more of antibiotic solutions are different and define a range, (b) incubating the microorganisms in the one or more of samples with an infectious agent comprising an indicator gene, and wherein the infectious agent is specific for the microorganism of interest, and (c) detecting an indicator protein product produced by the infectious agent in the one or more of samples, wherein detection of the indicator protein product in one or more of the plurality of samples indicates the concentrations of antibiotic solutions used to treat the one or more of the one or more of samples are not effective, and the lack of detection of the indicator protein indicates the antibiotic is effective, thereby determining the effective dose of the antibiotic.

The antibiotic and the infectious agent can be simultaneously added to the sample such that the sample contacts with both antibiotic and the infectious agent. The antibiotic and the infectious agent can be added sequentially, e.g., the sample is contacted with the antibiotic before the sample is contacted with the infectious agent. The method comprises incubating the sample with the antibiotic for a period time before contacting the sample with the infectious agent. The incubation time may vary depending on the nature of the antibiotic and the microorganism, for example based on the doubling time of the microorganism. In some embodiments, the incubation time is less than 24 hours, less than 18 hours, less than 12 hours, less than 6 hours, less than 5 hours, less than 4 hours, less than 3 hours, less than 2 hours, less than 1 hour, less than 45 min, less than 30 min, less than 15 min, less than 10 min or less than 5 min. The incubation time of microorganism with the infectious agent may also vary depending on the life cycle of the particular infectious agent, in some cases, the incubation time is less than 4 hours, less than 3 hours, less than 2 hours, less than 1 hour, less than 45 min, less than 30 min, less than 15 min, less than 10 min or less than 5 min. Microorganisms that are resistant to the antibiotic will survive and may multiply, and the infectious agent that is specific to the microorganism will replicate; conversely, microorganisms that are sensitive to the antibiotic will be killed and thus the infectious agent will not replicate. The infectious agent according to this method comprises an indicator moiety, the amount of which corresponds to the amount of the microorganisms present in the sample that have been treated with the antibiotic. Accordingly, a positive detection of the indicator moiety indicates the microorganism is resistant to the antibiotic.

In some embodiments, the methods may be used to determine whether an antibiotic-resistant microorganism is present in a clinical sample. For example, the methods may be used to determine whether a patient is infected with *Staphylococcus aureus* that are resistant or susceptible to a particular antibiotic. A clinical sample obtained from a patient may then be incubated with an antibiotic specific for *S. aureus.* The sample may then be incubated with recombinant phage specific for *S. aureus* for a period of time. In samples with *S. aureus* resistant to the antibiotic, detection of the indicator protein produced by the recombinant phage will be positive. In samples with *S. aureus* susceptible to the antibiotic, detection of the indicator protein will be negative. In some embodiments, methods for detection of antibiotic resistance may be used to select an effective therapeutic to which the pathogenic bacteria is susceptible.

In certain embodiments the total time required for detection is less than 6.0 hours, 5.0 hours, 4.0 hours, 3.0 hours, 2.5 hours, 2.0 hours, 1.5 hours, or less than 1.0 hour. The total time required for detection will depend on the bacteria of interest, the type of phage, and antibiotic being tested.

Optionally, the method further comprises lysing the microorganism before detecting the indicator moiety. Any solution that can lyse the microorganism can be used. In some cases, the lysis buffer may contain non-ionic detergents, chelating agents, enzymes or proprietary combinations of various salts and agents. Lysis buffers are also commercially available from Promega, Sigma-Aldrich, or Thermo-Fisher. Experiments suggest that infected unlysed cells may be detectable upon addition of luciferase substrate in some embodiments. Presumably, luciferase may exit cells and/or luciferase substrate may enter cells without complete cell lysis. Thus, for embodiments utilizing the spin filter system, where only luciferase released into the lysate (and not luciferase still inside intact bacteria) is analyzed in the luminometer, lysis is required for detection. However, for embodiments utilizing filter plates or 96-well plates with phage-infected sample in solution or suspension as described below, where intact and lysed cells may be directly assayed in the luminometer, lysis may not be necessary for detection. Thus, in some embodiments, the method of detecting antibiotic resistance does not involve lysing the microorganism.

A surprising aspect of the assays is that the step of incubating the microorganism in a sample with infectious agent only needs to be long enough for a single life cycle of the infectious agent, e.g., a phage. The amplification power of using phage was previously thought to require more time, such that the phage would replicate for several cycles. A single replication of indicator phage may be sufficient to facilitate sensitive and rapid detection according to some embodiments of the present invention. Another surprising aspect of the assays is that high concentrations of phage utilized for infecting test samples (i.e., high MOI) have successfully achieved detection of very low numbers of antibiotic resistant target microorganisms that have been treated with antibiotic. Factors, including the burst size of the phage, can affect the number of phage life cycles, and therefore, amount of time needed for detection. Phage with a large burst size (approximately 100 PFU) may only require one cycle for detection, whereas phage with a smaller burst size (e.g., 10 PFU) may require multiple phage cycles for detection. In some embodiments, the incubation of phage with a test sample need only be long enough for a single phage life cycle. In other embodiments, the incubation of phage with a test sample is for an amount of time greater than a single life cycle. The phage concentration for the incubating step will vary depending on the type of phage used. In some embodiments, the phage concentration for this incubating step is greater than 1.0 x 10⁵, greater than 1.0 x 10⁶, greater than 1.0 x 10⁷, or greater than 1.0 x 10⁸ PFU/mL. Success with such high concentrations of phage is surprising because such large numbers of phage were previously associated with "lysis from without," which killed target cells immediately and thereby prevented generation of useful signal from earlier phage assays. It is possible that the purification of the phage stock described herein helps to alleviate this problem (e.g., purification by cesium chloride isopycnic density gradient ultracentrifugation), because in addition to removing any contaminating luciferase associated with the phage, this purification may also remove ghost particles (particles that have lost DNA). The ghost particles can lyse bacterial cells via "lysis from without," killing the cells prematurely and thereby preventing generation of indicator signal. Electron microscopy demonstrates that a crude recombinant phage lysate (i.e., before cesium chloride purification) may have greater than 50% ghosts. These ghost particles may contribute to premature death of the microorganism through the action of many phage particles puncturing the cell membrane. Thus, ghost particles may have contributed to previous problems where high PFU concentrations were reported to be detrimental.

Any of the indicator moieties as described in this disclosure may be used for detecting the viability of microorganisms after antibiotic treatment, thereby detecting antibiotic resistance. In some embodiments, the indicator moiety associated with the infectious agent may be detectable during or after replication of the infectious agent. For example, as described above, in some cases, the indicator moiety may be a protein that emits an intrinsic signal, such as a fluorescent protein (e.g., green fluorescent protein or others). The indicator may generate light and/or may be detectable by a color change. In some embodiments, a luminometer may be used to detect the indicator (e.g., luciferase). However, other machines or devices may also be used. For example, a spectrophotometer, CCD camera, or CMOS camera may detect color changes and other light emissions.

In some embodiments, exposure of the sample to antibiotic may continue for 5 minutes or more and detection at various time points may be desirable for optimal sensitivity. For example, aliquots of a primary sample treated with antibiotic can be taken at different time intervals (e.g. at 5 minutes, 10 minutes, or 15 minutes). Samples from varying time interval may then be infected with phage and indicator moiety measured following the addition of substrate.

In some embodiments, detection of the signal is used to determine antibiotic resistance. In some embodiments, the signal produced by the sample is compared to an experimentally determined value. In further embodiments, the experimentally determined value is a signal produced by a control sample In some embodiments, the background threshold value is determined using a control without microorganisms. In some embodiments, the experimentally determined value is a background threshold value calculated from an average background signal plus standard deviation of 1-3 times the average background signal, or greater. In some embodiments, the background threshold value may be calculated from average background signal plus standard deviation of 2 times the average background signal. In other embodiments, the background threshold value may calculated from the average background signal times some multiple (e.g., 2 or 3). Detection of a sample signal greater than the background threshold value indicates the presence of one or more antibiotic-resistant microorganisms in the sample. For example, the average background signal may be 250 RLU. The threshold background value may be calculated by multiplying the average background signal (e.g., 250) by 3 to calculate a value of 750 RLU. Samples with bacteria having a signal value greater than 750 RLU are determined to be positive for containing antibiotic-resistant bacteria.

Alternatively, the experimentally determined value is the signal produced by a control sample. Assays may include various appropriate control samples. For example, samples containing no infectious agent that is specific to the microorganism, or samples containing infectious agents but without microorganism, may be assayed as controls for background signal levels. In some cases, samples containing the microorganisms that have not been treated with the antibiotic, are assayed as controls for determining antibiotic resistance using the infectious agents.

In some embodiments, the sample signal is compared to the control signal to determine whether antibiotic-resistant microorganisms are present in the sample. Unchanged detection of the signal as compared to a control sample that is contacted with the infectious agent but not with the antibiotic indicates the microorganism is resistant to the antibiotic, and reduced detection of the indicator moiety as compared to a control sample that is contacted with infectious agent but not with antibiotic indicates the microorganism is susceptible to the antibiotic. Unchanged detection refers to the detected signal from a sample that has been treated with the antibiotic and infectious agent is at least 80%, at least 90%, or at least 95% of signal from a control sample that has not been treated with the antibiotic. Reduced detection refers to the detected signal from a sample that has been treated with the antibiotic and infectious agent is less than 80%, less than 70%, less than 60%, less than 50%, less than 40%, or at least 30% of signal from a control sample that has not been treated with the antibiotic.

Optionally, the sample comprising the microorganism of interest is an uncultured sample. Optionally, the infectious agent is a phage and comprises an indicator gene inserted into a late gene region of the phage such that expression of the indicator gene during phage replication following infection of host bacteria results in a soluble indicator protein product. Features of each of the compositions used in the methods, as described above, can be also be utilized in the methods for detecting antibiotic resistance of the microorganism of interest.

Also provided herein is a method of determining the effective dose of an antibiotic for killing a microorganism. In some embodiments, the antibiotic is effective at killing *Staphylococcus* species. For example, the antibiotic may be cefoxitin, which is effective against most methicillin-sensitive *S. aureus* (MSSA). Typically, one or more antibiotic solutions having different concentrations are prepared such that the different concentrations of the solutions define a range. In some cases, the concentration ratio of the least concentrated antibiotic solution to the most concentrated antibiotic solution ranges from 1:2 to 1:50, e.g., from 1:5 to 1:30, or from 1: 10 to 1:20. In some cases, the lowest concentration of the one or more antibiotic solution is at least 1 µg/mL, e.g., at least 2 µg/mL, at least 5 µg/mL at least 10 µg/mL, at least 20 µg/mL, at least 40 µg/mL, at least 80 µg/mL, or at least 100 µg/mL. Each of the one or more antibiotic solutions is incubated with one aliquot of the sample comprising the microorganism of interest. In some cases, the infectious agent that is specific to the microorganism and comprises an indicator moiety is added simultaneously with the antibiotic solutions. In some cases, the aliquots of sample are incubated with the antibiotic solutions for a period of time before the addition of the infectious agent. The indicator moiety can be detected, and positive detection indicates that the antibiotic solution is not effective and negative detection indicates the antibiotic solution is effective and the concentration of the antibiotic solution is an effective dose. Accordingly, in some embodiments, the method of determining effective dose of an antibiotic in killing a microorganism of interest comprises incubating each of one or more antibiotic solutions separately with a microorganism of interest in a sample, wherein the concentrations of the one or more antibiotic solutions are different and define a range; incubating the microorganism in the one or more samples with an infectious agent comprising an indicator moiety; detecting the indicator moiety of the infectious agent in the one or more samples, wherein positive detection of the indicator moiety in one or more of the one or more samples indicates the concentrations of antibiotic solutions used to treat the one or more of the one or more samples are not effective, and the lack of detection of the indicator protein indicates the antibiotic is effective, thereby determining the effective dose of the antibiotic. In some embodiments, two or more antibiotic solutions are tested and the concentration ratio of the least concentrated solution and the most concentrated solution in the one or more antibiotic solutions ranges from 1:2 to 1:50, e.g., from 1:5 to 1:30, or from 1: 10 to 1:20. In some cases, the lowest concentration of the one or more antibiotic solution is at least 1 µg/mL, e.g., at least 2 µg/mL, at least 5 µg/mL at least 10 µg/mL, at least 20 µg/mL, at least 40 µg/mL, at least 80 µg/mL, or at least 100 µg/mL.

Detection of antibiotic-resistant bacteria can be used to prevent the spread of infection in healthcare settings. Patients in a healthcare setting may be monitored for colonization of antibiotic-resistant bacteria. Preventative measures may then be implemented to prevent the spread of antibiotic-resistant bacteria.

Thus, aspects of the present invention provide methods for detection of microorganisms in a test sample via an indicator moiety. The indicator moiety is associated with an indicator bacteriophage. The indicator moiety may react with a substrate to emit a detectable signal or may emit an intrinsic signal (e.g., fluorescent protein). In some embodiments, the detection sensitivity can reveal the presence of as few as 50, 20, 10, 9, 8, 7, 6, 5, 4, 3, or 2 cells of the microorganism of interest in a test sample. In some embodiments, even a single cell of the microorganism of interest may yield a detectable signal. In some embodiments, the bacteriophage is a T4-like or ViI-like bacteriophage. The recombinant *Staphylococus-*specific bacteriophage is highly specific for methicillin-resistant *Staphylococcus aureus*(MRSA).

In some embodiments, the indicator moiety encoded by the infectious agent may be detectable during or after replication of the infectious agent. Many different types of detectable biomolecules suitable for use as indicator moieties are known in the art, and many are commercially available. In some embodiments the indicator phage comprises an enzyme, which serves as the indicator moiety. In some embodiments, the genome of the indicator phage is modified to encode a soluble protein. In some embodiments, the indicator phage encodes a detectable enzyme. The indicator may emit light and/or may be detectable by a color change. Various appropriate enzymes are commercially available, such as alkaline phosphatase (AP), horseradish peroxidase (HRP), or luciferase (Luc). In some embodiments, these enzymes may serve as the indicator moiety. In some embodiments, Firefly luciferase is the indicator moiety. In some embodiments, Oplophorus luciferase is the indicator moiety. In some embodiments, NANOLUC^{®} is the indicator moiety. Other engineered luciferases or other enzymes that generate detectable signals may also be appropriate indicator moieties.

Thus, in some embodiments, the recombinant bacteriophage of the methods is prepared from wild-type *Staphylococcus-specific* bacteriophage. In some embodiments, the indicator gene encodes a protein that emits an intrinsic signal, such as a fluorescent protein (e.g., green fluorescent protein or others). The indicator may emit light and/or may be detectable by a color change. In some embodiments, the indicator gene encodes an enzyme (e.g., luciferase) that interacts with a substrate to generate signal. In some embodiments, the indicator gene is a luciferase gene. In some embodiments, the luciferase gene is one of Oplophorus luciferase, Firefly luciferase, Renilla luciferase, External Gaussia luciferase, Lucia luciferase, or an engineered luciferase such as NANOLUC^{®}, Rluc8.6-535, or Orange Nano-lantern.

Detecting the indicator may include detecting emissions of light. In some embodiments, a luminometer may be used to detect the reaction of indicator (e.g., luciferase) with a substrate. The detection of RLU can be achieved with a luminometer, or other machines or devices may also be used. For example, a spectrophotometer, CCD camera, or CMOS camera may detect color changes and other light emissions. Absolute RLU are important for detection, but the signal to background ratio also needs to be high (e.g., > 2.0, > 2.5, or > 3.0) in order for single cells or low numbers of cells to be detected reliably.

In some embodiments, the indicator phage is genetically engineered to contain the gene for an enzyme, such as a luciferase, which is only produced upon infection of bacteria that the phage specifically recognizes and infects. In some embodiments, the indicator moiety is expressed late in the viral life cycle. In some embodiments, as described herein, the indicator is a soluble protein (e.g., soluble luciferase) and is not fused with a phage structural protein that limits its copy number.

Thus in some embodiments utilizing indicator phage, the invention comprises a method for detecting a microorganism of interest comprising the steps of capturing at least one sample bacterium; incubating the at least one bacterium with a plurality of indicator phage; allowing time for infection and replication to generate progeny phage and express soluble indicator moiety; and detecting the progeny phage, or preferably the indicator, wherein detection of the indicator demonstrates that the bacterium is present in the sample.

For example, in some embodiments the test sample bacterium may be captured by binding to the surface of a plate, or by filtering the sample through a bacteriological filter (e.g., 0.45 µm pore size spin filter or plate filter). In an embodiment, the infectious agent (e.g., indicator phage) is added in a minimal volume to the captured sample directly on the filter. In an embodiment, the microorganism captured on the filter or plate surface is subsequently washed one or more times to remove excess unbound infectious agent. In an embodiment, a medium (e.g., Luria-Bertani Broth, also called LB herein, or Tryptic Soy Broth or Tryptone Soy Broth, also called TSB herein) may be added for further incubation time, to allow replication of bacterial cells and phage and high-level expression of the gene encoding the indicator moiety. However, a surprising aspect of some embodiments of testing assays is that the incubation step with indicator phage only needs to be long enough for a single phage life cycle. The amplification power of using bacteriophage was previously thought to require more time, such that the phage would replicate for several cycles. A single replication cycle of indicator phage can be sufficient to facilitate sensitive and rapid detection according to some embodiments of the present invention.

In some embodiments, aliquots of a test sample comprising bacteria may be applied to a spin column and after infection with a recombinant bacteriophage and an optional washing to remove any excess bacteriophage, the amount of soluble indicator detected will be proportional to the amount of bacteriophage that are produced by infected bacteria.

Soluble indicator (e.g., luciferase) released into the surrounding liquid upon lysis of the bacteria may then be measured and quantified. In an embodiment, the solution is spun through the filter, and the filtrate collected for assay in a new receptacle (e.g., in a luminometer) following addition of a substrate for the indicator enzyme (e.g., luciferase substrate). Alternatively, the indicator signal may be measured directly on the filter.

In various embodiments, the purified parental indicator phage does not comprise the detectable indicator itself, because the parental phage can be purified before it is used for incubation with a test sample. Expression of late (Class III) genes occurs late in the viral life cycle. In some embodiments of the present invention, parental phage may be purified to exclude any existing indicator protein (e.g., luciferase). In some embodiments, expression of the indicator gene during bacteriophage replication following infection of host bacteria results in a soluble indicator protein product. Thus, in many embodiments, it is not necessary to separate parental from progeny phage prior to the detecting step. In an embodiment, the microorganism is a bacterium and the indicator phage is a bacteriophage. In an embodiment, the indicator moiety is soluble luciferase, which is released upon lysis of the host microorganism.

Thus, in an alternate embodiment, the indicator substrate (e.g., luciferase substrate) may be incubated with the portion of the sample that remains on a filter or bound to a plate surface. Accordingly, in some embodiments the solid support is a 96-well filter plate (or regular 96-well plate), and the substrate reaction may be detected by placing the plate directly in the luminometer.

For example, in an embodiment, the invention may comprise a method for detecting MRSA comprising the steps of: infecting cells captured on a 96-well filter plate with a plurality of parental indicator phage capable of expressing luciferase upon infection; washing away excess phage; adding LB broth with antibiotic and allowing time for phage to replicate and lyse the specific *Staphylococcus aureus* target (e.g., 30-90 minutes); and detecting the indicator luciferase by adding luciferase substrate and measuring luciferase activity directly in the 96-well plate, wherein detection of luciferase activity indicates that the MRSA is present in the sample.

In some embodiments, lysis of the bacterium may occur before, during, or after the detection step. Experiments suggest that infected unlysed cells may be detectable upon addition of luciferase substrate in some embodiments. Presumably, luciferase may exit cells and/or luciferase substrate may enter cells without complete cell lysis. Thus, for embodiments utilizing the spin filter system, where only luciferase released into the lysate (and not luciferase still inside intact bacteria) is analyzed in the luminometer, lysis is required for detection. However, for embodiments utilizing filter plates or 96-well plates with sample in solution or suspension, where the original plate full of intact and lysed cells is directly assayed in the luminometer, lysis is not necessary for detection.

In some embodiments, the reaction of indicator moiety (e.g., luciferase) with substrate may continue for 30 minutes or more, and detection at various time points may be desirable for optimizing sensitivity. For example, in embodiments using 96-well filter plates as the solid support and luciferase as the indicator, luminometer readings may be taken initially and at 10- or 15-minute intervals until the reaction is completed.

Surprisingly, high concentrations of phage utilized for infecting test samples have successfully achieved detection of very low numbers of target microorganism in a very short timeframe. The incubation of phage with a test sample in some embodiments need only be long enough for a single phage life cycle. In some embodiments, the bacteriophage concentration for this incubating step is greater than 7 x 10⁶, 8 x 10⁶, 9 x 10⁶, 1.0 x 10⁷, 1.1 x 10⁷, 1.2 x 10⁷, 1.3 x 10⁷, 1.4 x 10⁷, 1.5 x 10⁷, 1.6 x 10⁷, 1.7 x 10⁷, 1.8 x 10⁷, 1.9 x 10⁷, 2.0 x 10⁷, 3.0 x 10⁷, 4.0 x 10⁷, 5.0 x 10⁷, 6.0 x 10⁷, 7.0 x 10⁷, 8.0 x 10⁷, 9.0 x 10⁷, or 1.0 x 10⁸ PFU/mL.

Success with such high concentrations of phage is surprising because the large numbers of phage were previously associated with "lysis from without," which killed target cells and thereby prevented generation of useful signal from earlier phage assays. It is possible that the clean-up of prepared phage stocks described herein helps to alleviate this problem (e.g., clean-up by cesium chloride isopycnic density gradient ultracentrifugation), because in addition to removing any contaminating luciferase associated with the phage, this clean-up may also remove ghost particles (particles that have lost DNA). The ghost particles can lyse bacterial cells via "lysis from without," killing the cells prematurely and thereby preventing generation of indicator signal. Electron microscopy demonstrates that a crude phage lysate (i.e., before cesium chloride clean-up) may have greater than 50% ghosts. These ghost particles may contribute to premature death of the microorganism through the action of many phage particles puncturing the cell membrane. Thus ghost particles may have contributed to previous problems where high PFU concentrations were reported to be detrimental. Moreover, a very clean phage prep allows the assay to be performed with no wash steps, which makes the assay possible to perform without an initial concentration step. Some embodiments do include an initial concentration step, and in some embodiments this concentration step allows a shorter enrichment incubation time.

Some embodiments of testing methods may further include confirmatory assays. A variety of assays are known in the art for confirming an initial result, usually at a later point in time. For example, the samples can be cultured (e.g., CHROMAGAR^{®}/DYNABEADS^{®} assay as described in Example 4), PCR can be utilized to confirm the presence of the microbial DNA, or other confirmatory assays can be used to confirm the initial result.

In certain embodiments, the methods of the present invention combine the use of a binding agent (e.g., antibody) to purify and/or concentrate a microorganism of interest from the sample in addition to detection with an infectious agent. For example, in certain embodiments, the present invention comprises a method for detecting a microorganism of interest in a sample comprising the steps of: capturing the microorganism from the sample on a prior support using a capture antibody specific to the microorganism of interest; incubating the sample with a recombinant bacteriophage that infects the microorganism of interest, wherein the recombinant bacteriophage comprises an indicator gene inserted into a late gene region of the bacteriophage such that expression of the indicator gene during bacteriophage replication following infection of host bacteria results in a soluble indicator protein product; and detecting the indicator protein product, wherein positive detection of the indicator protein product indicates that the microorganism of interest is present in the sample.

For example, **Figure 8** depicts a Hybrid Immuno-Phage (HIP) Assay for detecting a bacterium of interest using a modified bacteriophage according to an embodiment of the invention. The sample is first applied to the microtiter plate well coated with bacterium-specific antibodies **802.** The plate is then centrifuged to facilitate binding of the bacterium to the capture antibodies **804.** Following sufficient time to allow for complete bacteria capture, a solution containing bacterium-specific NANOLUC^{®}-phage is added to each sample **806.** Incubation with the phage results in the binding and attachment of a single or multiple phages to the captured bacterium **808.** Finally, the sample is incubated to facilitate phage replication and luciferase expression, which leads to cell lysis and release of soluble luciferase **810.**

Recent advances in synthetic biology have increased interest in bacteriophage-based therapeutic approaches to treating pathogenic disease (see, e.g., Phage Therapy in the Era of Synthetic Biology, Cold Spring Harb Perspect Biol 2016; 8:a023879; and U.S. Patent No. 9,597,407 and U.S. Patent App. Nos. 20170266306 and 20160331804). Bacteriophage designed and engineered to detect pathogens in medical samples from a patient for the presence of particular microbes, such as specific types of bacteria, can enhance the usefulness of such therapeutic phage.

Indicator phage can be employed to test initial patient samples for the presence of particular pathogens, such as a particular genus or species of bacterium. The indicator phage may be used to detect a particular pathogen in a clinical sample. In this way the indicator phage can be used in similar manner as a companion diagnostic, so as to evaluate the potential efficacy for specific therapies, such as particular antibiotics, other drugs, or therapeutic phages, e.g., in the context of a given patient's infection or other pathogenic medical condition. In some embodiments the diagnostic indicator phage can be prepared by genetic modification of naturally occurring bacteriophages as previously described.

In some embodiments, the indicator phage prepared through synthetic technologies may be used for non-clinical uses. For example, the indicator phage may be used as a food safety diagnostic to identify the presence of a particular bacteria in food. In other embodiments, the diagnostic indicator phage can be prepared through synthetic technologies. For example, a synthetic phage genome can be designed and constructed for transformation and propagation of corresponding phage in various types of bacteria. In some instances, the synthetic biology techniques can be used to generate an indicator phage using the indicator phage target bacteria. In other instances a more convenient bacteria can be used generate an indicator phage. For example, A synthetic genome for an indicator phage that targets *Listeria* may be generated in *Listeria,* or a more convenient species (e.g., *Lactobacillus*)*.*

In some embodiments synthetic phage are designed to optimize desirable traits for use in pathogen detection assays. In some embodiments bioinformatics and previous analyses of genetic modifications are employed to optimize desirable traits. For example, in some embodiments, the genes encoding phage tail proteins can be optimized to recognize and bind to particular species of bacteria. In other embodiments the genes encoding phage tail proteins can be optimized to recognize and bind to an entire genus of bacteria, or a particular group of species within a genus. In this way, the phage can be optimized to detect broader or narrower groups of pathogens. In some embodiments, the synthetic phage may be designed to improve expression of the reporter gene. Additionally and/or alternatively, in some instances, the synthetic phage may be designed to increase the burst size of the phage to improve detection.

In some embodiments, the stability of the phage may be optimized to improve shelf-life. For example, enzybiotic solubility may be increased in order to increase subsequent phage stability. Additionally and/or alternatively phage thermostability may be optimized. Thermostable phage better preserve functional activity during storage thereby increasing shelf-life. Thus, in some embodiments, the thermostability and/or pH tolerance may be optimized.

Some species of bacteria build biofilm walls to protect themselves against attacks by the immune system. These biofilms can make it difficult to effectively target bacteria. A number of enzymes (e.g., glycoside hydrolases PelAh and PslGh) have been identified that are capable of breaking down bacterial biofilm. In some embodiments, phage can be modified to code for either soluble or fusion virion proteins to allow incorporation of enzymes to break down biofilms.

In some embodiments the genetically modified phage or the synthetically derived phage comprises a detectable indicator. In some embodiments the indicator is a luciferase. The phage genome comprises an indicator gene (e.g., a luciferase gene or another gene encoding a detectable indicator).

In another aspect, the invention may comprise: a method for selecting a treatment for a subject comprising: (i) obtaining a biological sample from the subject; (ii) detecting a specific microorganism or category of microorganisms in the biological sample using an indicator phage; and (iii) selecting a treatment based on the identity of a specific microorganism detected in the biological sample.

In some embodiments the indicator phage, whether synthetically prepared or not, can be used to detect pathogens in patient samples subsequent to the initiation of some type of treatment. In some embodiments, the treatment can be a phage-based therapeutic. In other embodiments, the treatment can be an antibiotic (e.g., a traditional antibiotic such as penicillin or cyclosporine). In other embodiments, the treatment can be another type of drug or therapy. In this way the indicator phage can be used to monitor the progress or efficacy of any type of treatment or therapy. In some embodiments indicator phage can be used to detect and monitor the pathogenic content of patient samples taken hours or days after the initiation of treatment. In some embodiments indicator phage can be used to monitor samples in the context of a chronic infection and may be days, weeks, months, or years following the initiation of a treatment.

### EXAMPLES

Any examples not falling within the scope of the claims are for illustrative purposes only. Results depicted in the following examples demonstrate detection of a low number of cells, even a single bacterium, in a shortened time to results.

### Example 1. Bacterial Detection Using Staphylococcus aureus-Specific Bacteriophage NanoLuc Indicator Phage After Incubation of Sample in Media

Samples of methicillin-resistant *S. aureus* (MRSA) were primed by adding 135 µl of samples containing *S. aureus* to rich media. The rich media included sub-inhibitory antibiotic (cefoxitin) to allow for specific enrichment and induction of MRSA. Samples were incubated in rich media for 1-2 hours. Following the priming of the samples, additional cefoxitin was added to the media and samples were incubated for an additional 2 hours. *Staphylococcus aureus-*specific bacteriophage NanoLuc Indicator phage was added to the samples and incubated for 2 hours. Lysis buffer and NANO-GLO^{®} reagent were added. Following 5 minutes of incubation, measurements of bioluminescence were taken using a GLOMAX^{®} 96 instrument. A signal/background ratio ≥750 RLU indicated positive detection of *S. aureus.* A signal/background ratio of <750 RLU indicated the sample was negative for *S. aureus.*

**Table 1.1 Phage-Mediated Detection of Staphylococcus aureus**

| **Strain** | **Source** | **MR/MS** | **Detected CPU** | **RLU** | **Result** |
|---|---|---|---|---|---|
| **33592** | ATCC | MRSA | 140 | 1552 | Positive |
| **011-719** | Emory | MRSA | 146 | 6159 | Positive |
| **041-332** | Emory | MRSA | 152 | 32550 | Positive |
| **045-188** | Emory | MRSA | 115 | 1845 | Positive |
| **045-696** | Emory | MRSA | 444 | 38110 | Positive |
| **049-841** | Emory | MRSA | 134 | 4800 | Positive |
| **AR0461** | CDC Panel | MRSA | 159 | 16000 | Positive |
| **AR0462** | CDC Panel | MRSA | 72 | 616 | Negative |
| **AR0463** | CDC Panel | MRSA | 149 | 5058 | Positive |
| **AR0464** | CDC Panel | MRSA | 167 | 32430 | Positive |
| **AR0465** | CDC Panel | MRSA | 111 | 8831 | Positive |
| **AR0466** | CDC Panel | MRSA | 205 | 575 | Negative |
| **AR0467** | CDC Panel | MRSA | 198 | 7002 | Positive |
| **AR0468** | CDC Panel | MRSA | 203 | 29460 | Positive |
| **AR0469** | CDC Panel | MRSA | 21 | 3395 | Positive |
| **AR0469** | CDC Panel | MRSA | 287 | 30000 | Positive |
| **AR0469** | CDC Panel | MRSA | 473 | 19030 | Positive |
| **AR0470** | CDC Panel | MRSA | 314 | 2409 | Positive |
| **AR0471** | CDC Panel | MRSA | 219 | 584 | Negative |
| **AR0472** | CDC Panel | MRSA | 79 | 12720 | Positive |
| **AR0473** | CDC Panel | MRSA | 211 | 26910 | Positive |
| **AR0474** | CDC Panel | MRSA | 24 | 2888 | Positive |
| **AR0475** | CDC Panel | MRSA | 162 | 98540 | Positive |
| **AR0476** | CDC Panel | MRSA | 136 | 104400 | Positive |
| **AR0477** | CDC Panel | MRSA | 178 | 58230 | Positive |
| **AR0478** | CDC Panel | MRSA | 190 | 50920 | Positive |
| **AR0479** | CDC Panel | MRSA | 158 | 11460 | Positive |
| **AR0480** | CDC Panel | MRSA | 162 | 31600 | Positive |
| **AR0480** | CDC Panel | MRSA | 205 | 74630 | Positive |
| **AR0481** | CDC Panel | MRSA | 133 | 106300 | Positive |

**Table 1.2 Phage-Mediated Detection of Staphylococcus aureus**

| **Strain** | **Source** | **MR/MS** | **Detected CPU** | **RLU** | **Result** |
|---|---|---|---|---|---|
| **AR0482** | CDC Panel | MRSA | 134 | 452 | Negative |
| **AR0483** | CDC Panel | MRSA | 125 | 7415 | Positive |
| **BAA-1683** | ATCC | MRSA | 91 | 41560 | Positive |
| **SAA-1717** | ATCC | MRSA | 184 | 57700 | Positive |
| **BAA-1720** | ATCC | MRSA | 110 | 29120 | Positive |
| **BAA-1754** | ATCC | MRSA | 178 | 37590 | Positive |
| **BAA-2094** | ATCC | MRSA | 117 | 37280 | Positive |
| **BAA-2313** | ATCC | MRSA | 122 | 40430 | Positive |
| **BAA-41** | ATCC | MRSA | 110 | 25720 | Positive |
| **BAA-41** | ATCC | MRSA | 161 | 3916 | Positive |
| **BAA-42** | ATCC | MRSA | 111 | 4350 | Positive |
| **BAA-44** | ATCC | MRSA | 307 | 58850 | Positive |
| **USA300** | Emory | MRSA | 216 | 7325 | Positive |
| **6538** | ATCC | MSSA | 123 | 53340 | Positive |
| **12600** | ATCC | MSSA | 228 | 4472 | Positive |
| **14775** | ATCC | MSSA | 193 | 55190 | Positive |
| **25923** | ATCC | MSSA | 107 | 45600 | Positive |
| **27660** | ATCC | MSSA | 486 | 28160 | Positive |
| **29213** | ATCC | MSSA | 192 | 5820 | Positive |
| **502A** | ATCC | MSSA | 237 | 1427 | Positive |
| **AR0484** | CDC Panel | MSSA | 106 | 18540 | Positive |
| **AR0485** | CDC Panel | MSSA | 76 | 7876 | Positive |
| **AR0485** | CDC Panel | MSSA | 248 | 17100 | Positive |
| **AR0486** | CDC Panel | MSSA | 158 | 13010 | Positive |
| **AR0487** | CDC Panel | MSSA | 198 | 7182 | Positive |
| **AR0488** | CDC Panel | MSSA | 360 | 57240 | Positive |
| **AR0489** | CDC Panel | MSSA | 169 | 27370 | Positive |
| **AR0490** | CDC Panel | MSSA | 109 | 11900 | Positive |
| **ARD491** | CDC Panel | MSSA | 198 | 21290 | Positive |
| **AR0492** | CDC Panel | MSSA | 130 | 41150 | Positive |
| **BAA-1718** | ATCC | MSSA | 180 | 16710 | Positive |
| **BAA-1721** | ATCC | MSSA | 141 | 77000 | Positive |
| **RN4220** | U of Iowa | MSSA | 138 | 2500 | Positive |

**Table 1.3 Phage-Mediated Detection of Staphylococcus aureus**

| | | | | **Cefoxitin Concentration** | | | |
|---|---|---|---|---|---|---|---|
| **Strain** | **Source** | **MR/MS** | **Detected CFU** | **0 ug/mL** | **1 ug/mL** | **2 ug/mL** | **4 ug/mL** |
| **AR0461** | CDC Panel | MRSA | 25 | | 3460 | 1846 | 910 |
| **AR0463** | CDC Panel | MRSA | 265 | | 8987 | 3377 | 339 |
| **AR0465** | CDC Panel | MRSA | 101 | | 8263 | 4086 | 3006 |
| **AR0467** | CDC Panel | MRSA | 171 | | 9908 | 6497 | 4401 |
| **AR0468** | CDC Panel | MRSA | 169 | | 10330 | 10070 | 5002 |
| **AR0469** | CDC Panel | MRSA | 523 | | 35110 | 22030 | 9007 |
| **AR0470** | CDC Panel | MRSA | 215 | | 3502 | 1846 | 179 |
| **AR0472** | CDC Panel | MRSA | 142 | | 72720 | 19210 | 240 |
| **AR0473** | CDC Panel | MRSA | 219 | 76160 | 60000 | 17500 | 338 |
| **AR0474** | CDC Panel | MRSA | 477 | 15220 | 3449 | 2235 | 1684 |
| **AR0475** | CDC Panel | MRSA | 134 | 43580 | 52460 | 57660 | 34040 |
| **AR0476** | CDC Panel | MRSA | 95 | 384500 | 245000 | 52910 | 21 |
| **AR0477** | CDC Panel | MRSA | 471 | 1052000 | 478000 | 95970 | 4068 |
| **AR0478** | CDC Panel | MRSA | 183 | 394500 | 279700 | 73090 | 1519 |
| **AR0479** | CDC Panel | MRSA | 52 | 64460 | 51560 | 34730 | 6803 |
| **AR0480** | CDC Panel | MRSA | 356 | 1046000 | 1038000 | 430200 | 127400 |
| **AR0481** | CDC Panel | MRSA | 82 | 144300 | 150100 | 109000 | 27170 |
| **AR0482** | CDC Panel | MRSA | 75 | 10220 | 6741 | 2477 | 204 |
| **AR0483** | CDC Panel | MRSA | 12 | 31200 | 39250 | 24390 | 9081 |
| **BAA-2094** | ATCC | MRSA | 147 | | 252900 | 96700 | 1971 |
| **BAA-2313** | ATCC | MRSA | 192 | | 363300 | 52440 | 2409 |
| **BAA-42** | ATCC | MRSA | 165 | | 8634 | 775 | 240 |

**Table 1.4 Phage-Mediated Detection of Staphylococcus aureus**

| | | | | **Cefoxitin Concentration** | | | |
|---|---|---|---|---|---|---|---|
| **Strain** | **Source** | **MR/MS** | **Detected CPU** | **0 ug/mL** | **1 ug/mL** | **2 ug/mL** | **4 ug/mL** |
| **12600** | ATCC | MSSA | 406 | | 18310 | 178 | 196 |
| **AR0484** | CDC Panel | MSSA | 525 | 1902000 | 8875 | 172 | 182 |
| **AR0485** | CDC Panel | MSSA | 190 | | 13930 | 236 | 301 |
| **AR0486** | CDC Panel | MSSA | 220 | | 9217 | 236 | 252 |
| **AR0487** | CDC Panel | MSSA | 221 | | 10420 | 167 | 188 |
| **AR0488** | CDC Panel | MSSA | 151 | 259700 | 15430 | 207 | 197 |
| **AR0489** | CDC Panel | MSSA | 144 | 56600 | 30590 | 188 | 207 |
| **AR0490** | CDC Panel | MSSA | 940 | 910000 | 40620 | 254 | 582 |
| **AR0491** | CDC Panel | MSSA | 217 | 193100 | 40760 | 215 | 266 |
| **AR0492** | CDC Panel | MSSA | 178 | 288400 | 13040 | 194 | 207 |
| **BAA-1721** | ATCC | MSSA | 197 | | 21900 | 172 | 164 |

**Table 1.5 Phage-Mediated Detection of Staphylococcus aureus**

| | | | | **Cefoxitin Concentration** | |
|---|---|---|---|---|---|
| **Strain** | **Source** | **Detected CPU** | **MR/MS** | **0 ug/mL** | **1.8 ug/mL** |
| **BAA-1720** | ATCC | 16 | MRSA | 3335 | 4034 |
| **12600** | ATCC | 59 | MSSA | 12470 | 508 |
| **AR0463** | CDC Panel | 28 | MRSA | 4339 | 1941 |
| **AR0472** | CDC Panel | 14 | MRSA | 2948 | 2694 |

### Example 2. Diagnosis and Monitoring of Staphylococcus Infection Using Staphylococcus-specific bacteriophage NanoLuc^{®} Indicator Phage after incubation of sample in media

Obtain a clinical sample from a patient. Incubate the clinical sample with *Staphylococcus-specific* bacteriophage NanoLuc^{®} indicator phage for 2 hours. Following incubation, add lysis buffer and NANO-GLO^{®} reagent. Following 5 minutes of incubation with lysis buffer and NANO-GLO^{®} reagent, measure bioluminescence using a GLOMAX^{®} 96 instrument to determine the presence of *Staphylococcus* in the clinical sample. A signal/background ratio ≥750 RLU indicates positive detection of *Staphylococcus.* A signal/background ratio of <750 RLU indicates the sample is negative for *Staphylococcus.*

Patients with samples positive for *Staphylococcus* are treated with a therapeutic phage specific for *Staphylococcus.* Following treatment with a therapeutic phage specific for *Staphylococcus* for 72 hours, a second clinical sample is obtained from the patient. The clinical sample is then incubated with *Staphylococcus-specific* bacteriophage NanoLuc^{®} indicator phage for 2 hours. Following incubation, add lysis buffer and NANO-GLO^{®} reagent and incubate for 5 minutes. Then, measure bioluminescence using a GLOMAX^{®} 96 instrument to monitor changes in the presence of *Staphylococcus* in clinical samples following treatment. A signal/background ratio ≥750 RLU indicates positive detection of *Staphylococcus.* A signal/background ratio of <750 RLU indicates the sample is negative for *Staphylococcus.* If the sample is positive for *Staphylococcus,* continue treatment with a therapeutic phage specific for *Staphylococcus* and continue to monitor the efficacy of the treatment using the *Staphylococcus-*specific bacteriophage NanoLuc^{®} indicator phage.

## Claims

1. A method for selecting a treatment for a subject comprising:
(i) providing a biological sample obtained from the subject;
(ii) detecting a specific microorganism or category of microorganisms in the biological sample using an indicator phage wherein the indicator phage infects methicillin-resistant *Staphylococcus aureus*(*MRSA*) and the detection comprises the steps of: (a) incubating the sample with at least one antibiotic to allow for enrichment of bacteria that are resistant to the antibiotic, (b) incubating the enriched samples with bacteriophage that infects the bacterium of interest, wherein the bacteriophage comprises an indicator gene such that expression of the indicator gene during bacteriophage replication following infection of the bacterium of interest results in production of a soluble indicator protein product; and (c) detecting the indicator protein product, wherein positive detection of the indicator protein product indicates that the antibiotic-resistant bacterium of interest is present in the sample; and
(iii) selecting a treatment based on the identity of a specific microorganism detected in the biological sample.

2. The method of claim 1, wherein the indicator phage is a synthetically prepared phage.

3. The method of claim 1, wherein the indicator phage is a genetically modified naturally occurring phage.

4. A method for monitoring the efficacy of a treatment for a subject having a pathogenic medical condition comprising:
(i) providing a biological sample obtained from the subject;
(ii) detecting a specific microorganism or category of microorganisms in the biological sample using an indicator phage wherein the indicator phage infects methicillin-resistant Staphylococcus aureus (MRSA) and the detection comprises the steps of: (a) incubating the sample with at least one antibiotic to allow for enrichment of bacteria that are resistant to the antibiotic, (b) incubating the enriched samples with bacteriophage that infects the bacterium of interest, wherein the bacteriophage comprises an indicator gene such that expression of the indicator gene during bacteriophage replication following infection of the bacterium of interest results in production of a soluble indicator protein product; and (c) detecting the indicator protein product, wherein positive detection of the indicator protein product indicates that the antibiotic-resistant bacterium of interest is present in the sample;
(iii) after treatment of the subject,
providing a second biological sample of the same type as the first biological sample obtained from the subject;
(iv) detecting the specific microorganism or category of microorganisms in the second biological sample using the indicator phage; and
(v)
determining a decrease, increase, or steady level of the specific microorganism or category of microorganisms in the subject based on the amounts detected in the first and second biological samples.

5. The method of claim 4, wherein the indicator phage is a synthetically prepared phage.

6. The method of claim 4,
wherein the indicator phage is a genetically modified naturally occurring phage.

## Patentansprüche

1. Verfahren zum Auswählen einer Behandlung für ein Subjekt, umfassend:
(i) Bereitstellen einer biologischen Probe, die von dem Subjekt erhalten wird;
(ii) Nachweisen eines spezifischen Mikroorganismus oder einer Kategorie von Mikroorganismen in der biologischen Probe unter Verwendung eines Indikatorphagen, wobei der Indikatorphage Methicillin-resistente *Staphylococcus aureus* (MRSA) infiziert und der Nachweis die Schritte umfasst: (a) Inkubieren der Probe mit mindestens einem Antibiotikum, um eine Anreicherung von Bakterien zu ermöglichen, die gegen das Antibiotikum resistent sind; (b) Inkubieren der angereicherten Proben mit einem Bakteriophagen, der das Bakterium von Interesse infiziert, wobei der Bakteriophage ein Indikatorgen derart umfasst, dass eine Expression des Indikatorgens während einer Bakteriophagenreplikation nach der Infektion des Bakteriums von Interesse zu einer Produktion eines löslichen Indikatorproteinprodukts führt; und (c) Nachweisen des Indikatorproteinprodukts, wobei ein positiver Nachweis des Indikatorproteinprodukts anzeigt, dass das antibiotikaresistente Bakterium von Interesse in der Probe vorhanden ist; und
(iii) Auswählen einer Behandlung basierend auf der Identität eines spezifischen Mikroorganismus, der in der biologischen Probe nachgewiesen wird.

2. Verfahren nach Anspruch 1, wobei der Indikatorphage ein synthetisch hergestellter Phage ist.

3. Verfahren nach Anspruch 1, wobei der Indikatorphage ein genetisch veränderter natürlich vorkommender Phage ist.

4. Verfahren zum Überwachen der Wirksamkeit einer Behandlung für ein Subjekt, das einen pathogenen medizinischen Zustand aufweist, umfassend:
(i) Bereitstellen einer biologischen Probe, die von dem Subjekt erhalten wird;
(ii) Nachweisen eines spezifischen Mikroorganismus oder einer Kategorie von Mikroorganismen in der biologischen Probe unter Verwendung eines Indikatorphagen, wobei der Indikatorphage Methicillin-resistente *Staphylococcus aureus* (MRSA) infiziert und der Nachweis die Schritte umfasst: (a) Inkubieren der Probe mit mindestens einem Antibiotikum, um die Anreicherung von Bakterien zu ermöglichen, die gegen das Antibiotikum resistent sind; (b) Inkubieren der angereicherten Proben mit einem Bakteriophagen, der das Bakterium von Interesse infiziert, wobei der Bakteriophage ein Indikatorgen derart umfasst, dass die Expression des Indikatorgens während einer Bakteriophagenreplikation nach der Infektion des Bakteriums von Interesse zu einer Produktion eines löslichen Indikatorproteinprodukts führt; und (c) Nachweisen des Indikatorproteinprodukts, wobei ein positiver Nachweis des Indikatorproteinprodukts anzeigt, dass das antibiotikaresistente Bakterium von Interesse in der Probe vorhanden ist;
(iii) nach der Behandlung des Subjekts, Bereitstellen einer zweiten biologischen Probe desselben Typs wie die erste biologische Probe, die von dem Subjekt erhalten wird;
(iv) Nachweisen des spezifischen Mikroorganismus oder der Kategorie von Mikroorganismen in der zweiten biologischen Probe unter Verwendung des Indikatorphagen; und
(v) Bestimmen einer Abnahme, Zunahme oder eines gleichbleibenden Niveaus des spezifischen Mikroorganismus oder der Kategorie von Mikroorganismen in dem Subjekt basierend auf Mengen, die in der ersten und der zweiten biologischen Probe erkannt werden.

5. Verfahren nach Anspruch 4, wobei der Indikatorphage ein synthetisch hergestellter Phage ist.

6. Verfahren nach Anspruch 4, wobei der Indikatorphage ein genetisch veränderter natürlich vorkommender Phage ist.

## Revendications

1. Procédé de sélection d'un traitement pour un sujet comprenant :
(i) la fourniture d'un échantillon biologique prélevé sur le sujet ;
(ii) la détection d'un micro-organisme spécifique ou d'une catégorie de micro-organismes dans l'échantillon biologique à l'aide d'un phage indicateur, dans lequel le phage indicateur infecte le *Staphylococcus aureus* résistant à la méthicilline (SARM) et la détection comprenant les étapes consistant à : (a) incuber l'échantillon avec au moins un antibiotique pour permettre l'enrichissement de bactéries qui sont résistantes à l'antibiotique, (b) incuber des échantillons enrichis avec un bactériophage qui infecte la bactérie concernée, dans lequel le bactériophage comprend un gène indicateur tel que l'expression du gène indicateur pendant la réplication du bactériophage après l'infection de la bactérie concernée entraîne la production d'un produit protéique indicateur soluble ; et (c) détecter le produit protéique indicateur, dans lequel la détection positive du produit protéique indicateur indique la présence de la bactérie résistante aux antibiotiques dans l'échantillon ; et
(iii) sélectionner un traitement basé sur l'identité d'un micro-organisme spécifique détecté dans l'échantillon biologique.

2. Procédé selon la revendication 1, dans lequel le phage indicateur est un phage préparé synthétiquement.

3. Procédé selon la revendication 1, dans lequel le phage indicateur est un phage naturel génétiquement modifié.

4. Procédé de surveillance de l'efficacité d'un traitement pour un sujet souffrant d'un problème médical pathogène, comprenant :
(i) la fourniture d'un échantillon biologique prélevé sur le sujet ;
(ii) la détection d'un micro-organisme spécifique ou d'une catégorie de micro-organismes dans l'échantillon biologique à l'aide d'un phage indicateur, dans lequel le phage indicateur infecte le Staphylococcus aureus résistant à la méthicilline (SARM) et la détection comprenant les étapes consistant à : (a) incuber l'échantillon avec au moins un antibiotique pour permettre l'enrichissement de bactéries qui sont résistantes à l'antibiotique, (b) incuber des échantillons enrichis avec un bactériophage qui infecte la bactérie concernée, dans lequel le bactériophage comprend un gène indicateur tel que l'expression du gène indicateur pendant la réplication du bactériophage après l'infection de la bactérie concernée entraîne la production d'un produit protéique indicateur soluble ; et (c) détecter le produit protéique indicateur, dans lequel la détection positive du produit protéique indicateur indique la présence de la bactérie résistante aux antibiotiques dans l'échantillon ;
(iii) après le traitement du sujet, fournir un second échantillon biologique du même type que le premier échantillon biologique obtenu du sujet ;
(iv) détecter le micro-organisme spécifique ou la catégorie de micro-organismes dans le second échantillon biologique à l'aide du phage indicateur ; et
(v) déterminer une diminution, une augmentation ou un niveau stable du micro-organisme spécifique ou de la catégorie de micro-organismes chez le sujet sur la base des quantités détectées dans les premier et second échantillons biologiques.

5. Procédé selon la revendication 4, dans lequel le phage indicateur est un phage préparé synthétiquement.

6. Procédé selon la revendication 4, dans lequel le phage indicateur est un phage naturel génétiquement modifié.
